(19) Europäisches Patentamt
European Patent Office
Office européen des brevets

(11) EP 3 535 241 B1

(12) EUROPÄISCHE PATENTSCHRIFT

(45) Veröffentlichungstag und Bekanntmachung des Hinweises auf die Patenterteilung:
**03.12.2025 Patentblatt 2025/49**

(21) Anmeldenummer: **17801362.9**

(22) Anmeldetag: **01.11.2017**

(51) Internationale Patentklassifikation (IPC):
***C07C 317/18*** (2006.01) ***C07C 321/14*** (2006.01)
***C07F 9/09*** (2006.01) ***C11D 1/00*** (2006.01)

(52) Gemeinsame Patentklassifikation (CPC):
**C07C 323/12; C07C 317/04; C07C 317/18; C07C 323/66; C07F 9/091; C07F 9/65742; C11D 1/002; C11D 1/004; C11D 1/006**

(86) Internationale Anmeldenummer:
**PCT/EP2017/077946**

(87) Internationale Veröffentlichungsnummer:
**WO 2018/083110 (11.05.2018 Gazette 2018/19)**

(54) **FLUORTENSIDE**

FLUORTENSIDE

TENSIOACTIFS FLUORÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.11.2016 DE 102016013066**

(43) Veröffentlichungstag der Anmeldung:
**11.09.2019 Patentblatt 2019/37**

(73) Patentinhaber: **SUSONITY Commercial GmbH**
**64579 Gernsheim (DE)**

(72) Erfinder:
- **FRIEDRICH, Reiner**
**64342 Seeheim-Jugenheim (DE)**
- **KOCH, Fabian**
**64319 Pfungstadt (DE)**

(74) Vertreter: **Weckenbrock, Matthias**
**Dr. Müller Patentanwälte**
**Mühlstraße 9a**
**65597 Hünfelden (DE)**

(56) Entgegenhaltungen:
**WO-A1-2015/124290**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 535 241 B1

## Beschreibung

Gegenstand der vorliegenden Erfindung sind neue Verbindungen mit fluorierten Endgruppen, deren Verwendung als oberflächenaktive Substanzen und Mittel enthaltend diese Verbindungen.

Fluortenside stellen einen wichtigen Bestandteil in industriellen Prozesschemikalien dar. Aufgrund ihrer Persistenz und Toxizität sind diese Materialien problematisch für Anwender und Umwelt. Fluorhaltige Tenside sind in verschiedensten Anwendungen einsetzbar und tragen z.B. zur verbesserten Benetzung von Oberflächen bei. So werden sie z.B. als Grenzflächenvermittler bzw. Emulgator oder Viskositätsminderer in Farben, Lacken oder Klebstoffen verwendet. Klassische Fluortenside sind aus langkettigen, perfluorierten Alkylketten (C6-C8) aufgebaut und gelten potentiell als bioakkumulativ und toxisch. In der Regel enthalten Fluortenside jedoch Perfluoralkylsubstituenten, die in der Umwelt durch biologische und andere Oxidationsprozesse zu Perfluoralkancarbonsäuren und -sulfonsäuren abgebaut werden. Diese gelten als persistent und stehen z. T. im Verdacht gesundheitliche Schäden zu verursachen (G. L. Kennedy, Jr., J. L. Butenhoff, G. W. Olsen, J. C. O'Connor, A. M. Seacat, R. G. Perkins, L. B. Biegel, S. R. Murphy, D. G. Farrar, Critical Reviews in Toxicology 2004, 34, 351-384). Längerkettige Perfluoralkancarbonsäuren und -sulfonsäuren reichern sich zudem in der Nahrungskette an. Kürzerkettige Fluorbausteine sind von ihren ökotoxikologischen Profilen günstiger, zeigen jedoch in ihren Anwendungsbereichen oft schlechtere Eigenschaften. In WO 2006/072401 und WO 2010/003567 werden oberflächenaktive Verbindungen mit Trifluormethoxygruppen beschrieben. Weitere Fluortenside mit fluorierten Alkylgruppen, sind in WO 2009/149807, WO 2010/003567, WO 2010/149262, WO 2011/082770, WO 2012/084118, WO 2015/124290 und WO 2016/096129 beschrieben.

Weiterhin besteht Bedarf nach alternativen oberflächenaktiven Substanzen, die vorzugsweise beim Abbau nicht zu langkettigen persistenten Verbindungen abbauen. Es wurden nun neue Verbindungen gefunden, die als oberflächenaktive Substanzen geeignet sind und bevorzugt einen oder mehrere der o.g. Nachteile nicht aufweisen. Ein erster Gegenstand der vorliegenden Erfindung sind Verbindungen der Formeln (I), (IIa), (V), (VI), (VII), (XIII), (XIII') oder (XV) wie in Anspruch 1 definiert.

$$(R^1\text{-CHF-CF}_2\text{-Y-})_m\text{Spacer}(X)_n \qquad (I)$$

wobei in Formel (I)

$R^1$ = eine fluorierte, ggf. Heteroatome enthaltende, lineare oder verzweigte, Alkylgruppe ist,
Spacer = eine Einfachbindung oder eine bivalente organische Gruppe ist,
X = eine anionische, kationische, nicht-ionische oder amphotere hydrophile Gruppe,
Y = SO oder $SO_2$,
m = 1, 2, 3, 4, 5 oder 6
und n = 1, 2, 3 oder 4 ist,

Die erfindungsgemäßen Verbindungen enthalten bevorzugt keine -O-O-Bindungen. Die neuen Verbindungen der Formel (I) enthalten die folgenden Variablen:

$R^1$ = perfluoriertes Alkyl, linear oder verzweigt, ggf. Heteroatome enthaltend, bevorzugt perfluoriertes C1-C6-Alkyl, besonders bevorzugt perfluoriertes C1-C4-Alkyl, insbesondere perfluoriertes C1-C3-Alkyl,
Spacer = eine gesättigte oder ungesättigte, verzweigte oder unverzweigte, ggf. Heteroatome enthaltende, Kohlenwasserstoffeinheit, wobei keine -O-O-Bindungen vorliegen,
X = eine anionische, kationische, nicht-ionische oder amphotere hydrophile Gruppe,
Y = SO oder $SO_2$, bevorzugt S,
m = 1, 2, 3,4, 5 oder 6, bevorzugt 2-4, insbesondere 2-3, und
n = 1, 2, 3 oder 4, bevorzugt 1 oder 2.

Besondere bevorzugte Verbindungen der Formel (I) sind solche, in denen alle Variablen die bevorzugten Bedeutungen haben.

Bevorzugt ist die fluorierte Gruppe $R^1$ ausgewählt aus den Gruppen: $CF_3\text{-}(CF_2)_{0\text{-}3}\text{-}$, $CF_3\text{-}(CF_2)_{0\text{-}3}\text{-O-}$, $CF_3\text{-}(CF_2)_{0\text{-}3}\text{-O-}(CF_2)_{1\text{-}3}\text{-}$, $CF_3\text{-}(CF_2)_{0\text{-}3}\text{-O-}(CF_2)_{1\text{-}3}\text{-O-}$, $CF_3\text{-}(CF_2)_{0\text{-}3}\text{-O-}(CF_2)_{1\text{-}3}\text{-O-CF}_2\text{-}$, $CF_3\text{-}(CF_2)_{0\text{-}3}\text{O-}(CF_2\text{-O})_{1\text{-}8}\text{-}$ und $CF_3\text{-}(CF_2)_{0\text{-}3}\text{-O-}(CF_2\text{-O})_{1\text{-}8}\text{-CF}_2\text{-}$. Insbesondere bevorzugt ist die fluorierte Gruppe $R^1$ eine $CF_3\text{-}(CF_2)_{1\text{-}2}\text{-O}$-Gruppe, insbesondere eine $CF_3\text{-CF}_2\text{-CF}_2\text{-O}$-Gruppe.

Eine anionische Gruppe X ist ausgewählt aus $\text{-COO}^-$, $\text{-SO}_3^-$, $\text{-OSO}_3^-$, $\text{-PO}_3^{2-}$, $\text{-OPO}_3^{2-}$, $\text{-OP(O)(O}^-\text{)O-}$, $\text{-(OCH}_2\text{CH}_2)_s\text{-O-(CH}_2\text{)t-COO}^-$, $\text{-(OCH}_2\text{CH}_2)_s\text{-O-(CH}_2)_t\ \text{-SO}_3^-$, $\text{-(OCH}_2\text{CH}_2)_s\text{-O-(CH}_2)_t\text{-OSO}_3^-$, $\text{-(OCH}_2\text{CH}_2)_s\text{-O-(CH}_2)_t\text{-PO}_3^{2-}$, $\text{-(OCH}_2\text{CH}_2)_s\text{-O-(CH}_2)_t\text{-OPO}_3^{2-}$ oder aus den Formeln A bis C,

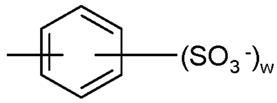

A

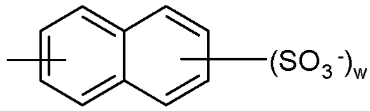

B

oder

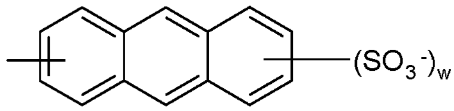

C

wobei s steht für eine ganze Zahl aus dem Bereich von 1 bis 1000, t steht für eine ganze Zahl ausgewählt aus 1, 2, 3 oder 4 und w steht für eine ganze Zahl ausgewählt aus 1, 2 oder 3.

Zu den bevorzugten anionischen Gruppen gehören dabei insbesondere $-COO^-$, $-SO_3^-$, $-OSO_3^-$, $-PO_3^{2-}$, $-OPO_3^{2-}$, $-OP(O)(O^-)O-$, die Teilformel A, sowie $-(OCH_2CH_2)_s-O-(CH_2)_t-COO^-$, $-(OCH_2CH_2)_s-O-(CH_2)_t-SO_3^-$ und $-(OCH_2CH_2)_s-O-(CH_2)_t-OSO_3^-$, wobei jede einzelne dieser Gruppen für sich genommen bevorzugt sein kann. X kann auch für die entsprechenden Säuren stehen.

Zu den ganz besonders bevorzugten anionischen Gruppen gehören dabei $-SO_3^-$, $-OSO_3^-$, $-COO^-$, $-PO_3^{2-}$, $-OP(O)(O^-)O-$ oder $-OPO_3^{2-}$. Insbesondere eine Sulfonatgruppe $-SO_3^-$ ist bevorzugt.

Das Gegenion für anionische Gruppen X ist ein einwertiges Kation, insbesondere $H^+$, ein Alkalimetall-Kation oder $NR_4^+$, wobei R = H oder C1 - C6-Alkyl ist und alle R gleich oder verschieden sein können. Insbesondere bevorzugt sind $H^+$, $Na^+$, $K^+$, $Li^+$ und $NH_4^+$, besonders bevorzugt $Na^+$.

Eine kationische Gruppe X ist ausgewählt aus

$-NR^1R^2R^{3+}\ Z^-$, $-PR^1R^2R^{3+}\ Z^-$,

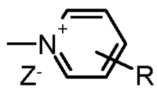
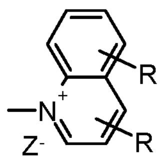
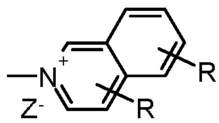

wobei R steht für H oder $C_{1-4}$-Alkyl in beliebiger Position,
$Z^-$ steht für $Cl^-$, $Br^-$, $I^-$, $CH_3SO_3^-$, $CF_3SO_3^-$, $CH_3PhSO_3^-$, $PhSO_3^-$
$R^1$, $R^2$ und $R^3$ jeweils unabhängig voneinander stehen für H, $C_{1-30}$-Alkyl, Ar oder $-CH_2Ar$ und
Ar steht für einen unsubstituierten oder ein- oder mehrfach substituierten aromatischen Ring oder kondensierte Ringsysteme mit 6 bis 18 C-Atomen, worin auch ein oder zwei CH-Gruppen durch N ersetzt sein können.

Zu den bevorzugten kationischen Gruppen gehören dabei insbesondere $-NR^1R^2R^{3+}\ Z^-$ und

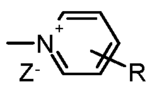

wobei jede einzelne dieser Gruppen für sich genommen bevorzugt sein kann.

Eine nicht-ionische Gruppe X ist ausgewählt aus: lineares oder verzweigtes Alkyl, wobei ein oder mehrere nicht benachbarte C-Atome durch O, S, und/oder N ersetzt sind, -OH, -SH, $-O-(Glycosid)_{o'}$, $-S-(Glycosid)_{o'}$, $-OCH_2-CHOH-CH_2-OH$, $-O\ CH_2Ar(-NCO)_{p'}$, $-OAr(-NCO)_{p'}$, Aminoxid,

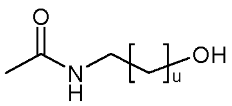 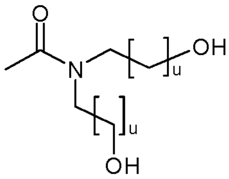 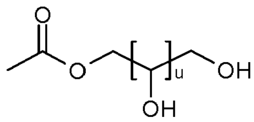

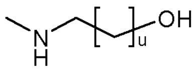 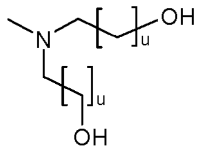 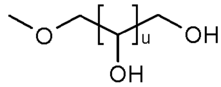

u steht für eine ganze Zahl aus dem Bereich von 1 bis 6, bevorzugt 1 bis 4
o' steht für eine ganze Zahl aus dem Bereich von 1 bis 10,
p' steht für 1 oder 2,
Ar steht für einen unsubstituierten, ein- oder mehrfach substituierten aromatischen Ring oder kondensierte Ringsysteme mit 6 bis 18 C-Atomen, worin auch ein oder zwei CH-Gruppen durch C=O ersetzt sein können und, Glycosid steht für ein verethertes Kohlenhydrat, vorzugsweise für ein mono- di-, tri- oder oligo-Glucosid.

Zu den bevorzugten nicht-ionischen Gruppen X gehören dabei insbesondere lineares oder verzweigtes Alkyl, wobei ein oder mehrere nicht benachbarte C-Atome durch O, S und/oder N ersetzt sind, -OH und -O-(Glycosid)$_{o'}$.

Wenn X = Alkyl, wobei ein oder mehrere nicht benachbarte C-Atome durch O, S, und/oder N ersetzt sind, dann ist es bevorzugt gleich $R^4$-(B-A)$_{m''}$- mit $R^4$ = H oder C1-4-Alkyl, insbesondere H oder $CH_3$, A = lineares oder verzweigtes Alkylen, bevorzugt mit 1 bis 10 Kohlenstoffatomen, insbesondere mit 1 bis 4 Kohlenstoffatomen, B = O oder S, bevorzugt O, und m" = eine ganze Zahl bevorzugt aus dem Bereich von 1 bis 100, besonders bevorzugt 1 bis 30.

Insbesondere bevorzugt als nicht-ionische Gruppe X ist die Gruppe $R^4$-(O-CH$_{2CHR}$$^5$)$_{m''}$- mit m" = eine ganze Zahl aus dem Bereich von 1 bis 100, bevorzugt 1 bis 30, insbesondere auch 1-25, und $R^4$ und $R^5$ = H oder C1-4-Alkyl, insbesondere H oder $CH_3$. Insbesondere bevorzugt ist $R^4$-(B-A)$_{m''}$- eine Polyethylen- oder Polypropylenglykoleinheit.

Insbesondere bevorzugt als nicht-ionische Gruppe X ist die Gruppe -CH(OH)-$CH_2$-NH-Sach mit Sach = verschiedene Zucker und die Gruppe -Y-($CH_2$-$CH_2$-O)$_v$-$R^4$ mit Y = S, O oder NH, bevorzugt O, $R^4$ = H oder Alkyl, bevorzugt H oder $CH_3$, und v = 1-100, bevorzugt 1-30, insbesondere auch 1-25.

Eine amphotere Gruppe X ist ausgewählt aus den funktionellen Gruppen der Acetyldiamine, der N-Alkylaminosäuren, der N-Alkylaminosulfonsäuren, der Betaine, der Sulfobetaine, bzw. entsprechender Derivate, insbesondere ausgewählt aus, wobei M steht für H oder ein Alkalimetall-Ion, vorzugsweise $Li^+$, $Na^+$ oder $K^+$:

| |
|---|
| 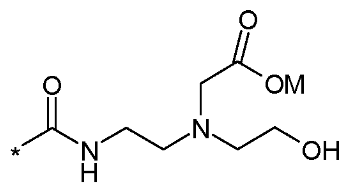 |
| 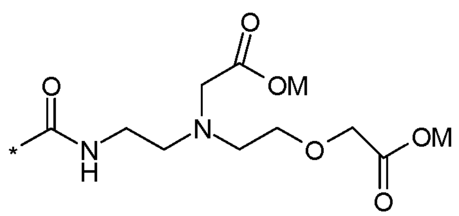 |

(fortgesetzt)

| |
|---|
| 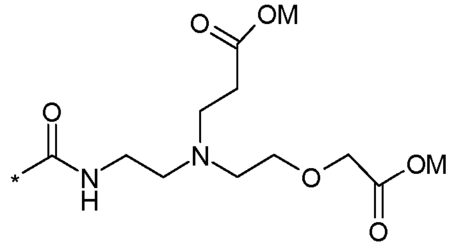 |
| $-NH-CH_2-COOM$; $-NH-CH_2-CH_2-COOM$ |
| 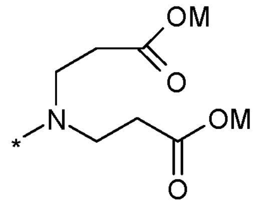 |
| $-[(C(=O)-NH-(CH_2)_{(1-8)}]_{(0\ oder\ 1)}-N^+R^1R^2-CH_2-COO^-$, wobei $R^1$ und $R^2$ jeweils unabhängig voneinander stehen für einen C1-8-Alkylrest, vorzugsweise Methyl oder Ethyl |
| $-C(=O)-NH-(CH_2)_{1-3}-N^+R^1R^2-CH_2-CH(OH)-CH_2-(O)_{(0\ oder\ 1)}-(S\ oder\ P)O_3^-$, wobei $R^1$ und $R^2$ jeweils unabhängig voneinander stehen für einen C1-8-Alkylrest, vorzugsweise Methyl oder Ethyl |

Besonders bevorzugte erfindungsgemäße Verbindungen sind solche, die als hydrophile Gruppe X eine der bevorzugten anionische Gruppen, der bevorzugten nicht-ionischen Gruppen oder der bevorzugten zwitterionischen Gruppen enthalten.

Insbesondere bevorzugt sind Verbindungen, die die Gruppen $-SO_3^-$, $-OSO_3$ , $-COO^-$, $-PO_3^{2-}$, $-OP(O)(O^-)O-$ oder $-OPO_3^{2-}$, Polyethylen- oder Polypropylenglykole, $-CH(OH)-CH_2-NH-Sach$, $-Y-(CH_2-CH_2-O)_v-R^4$, Betaine, oder Sulfobetaine enthalten. Bevorzugte Gegenionen sind hierbei $H^+$, $Na^+$, $K^+$ und $NH_4^+$, insbesondere $Na^+$. Insbesondere bevorzugt sind: $-SO_3^-$, $-COO^-$, $-OP(O)(O^-)O-$ oder $-OPO_3^{2-}$, Polyethylen- oder Polypropylenglykole, Sulfobetaine, die Gruppe $-CH(OH)-CH_2-NH-Sach$ und die Gruppe $-Y-(CH_2-CH_2-O)_v-R^4$. Hierbei ist Sach = verschiedene Zucker und Y = S, O oder NH, bevorzugt O, $R^4$ = H oder Alkyl, bevorzugt H oder $CH_3$, und v = 1-100, bevorzugt 1-30, insbesondere auch 1-25. Besonders vorteilhaft können auch Verbindungen mit X = $-SO_3^-$ sein.

Bei den Kohlenwasserstoff-Einheiten des Spacers der Verbindungen der Formel (I) kann es sich um aliphatische oder aromatische, gegebenenfalls mit Heteroatomen versehene Einheiten handeln. Vorzugsweise ist der Spacer eine gesättigte, verzweigte oder unverzweigte KohlenwasserstoffEinheit, bevorzugt eine gesättigte, verzweigte oder unverzweigte Alkylengruppe, wobei ein oder mehrere nicht benachbarte C-Atome durch O oder N, bevorzugt O, ersetzt oder mit O verbunden sein können. Bevorzugt sind z. B. C1-C6-Alkylengruppen, insbesondere C1-C4-Alkylengruppen.

In einer Erfindungsvariante wird als bevorzugte Heteroatome enthaltende Kohlenwasserstoffeinheit eine Polyethylen- oder Polypropylenglykoleinheit verwendet.

In den Verbindungen der Formeln (IIa-c) und (V) bedeuten $R^1$ und $R^2$ unabhängig voneinander eine fluorierte, ggf. Heteroatome enthaltende, lineare oder verzweigte, Alkylgruppe, o = 0-100, bevorzugt gleich 1-30 und 5-30, insbesondere 3, 5, 6, 10, 12, 15, 18, 20 oder 24, und $X^1$ und $X^2$ unabhängig voneinander eine anionische, kationische, nicht-ionische oder amphotere hydrophile Gruppe wie in Anspruch 1 definiert, bevorzugt eine der für X bevorzugten Gruppen, bzw. in den Formeln (IIa), (IIb), (IIc) und (V) auch gleich H:

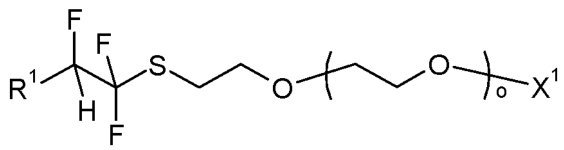

(IIa)

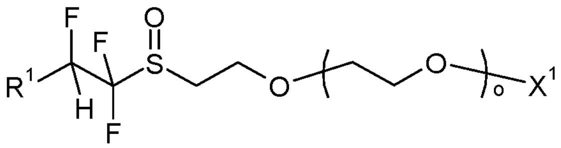

(IIb)

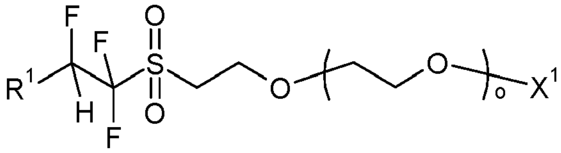

(IIc)

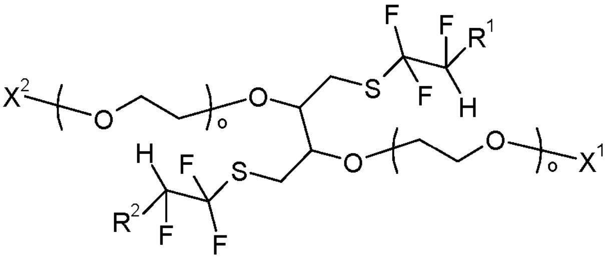

(V)

In einer bevorzugten Variante der Verbindungen der Formeln (IIa-c) und (V) sind $X^1$ und $X^2$ unabhängig voneinander eine anionische oder nicht-ionische Gruppe wie in Anspruch 1 definiert, insbesondere die für X bevorzugten Gruppen, und $R^1$ und $R^2$ unabhängig voneinander eine $CF_3$-$(CF_2)_{1-2}$-O- Gruppe. Bevorzugt sind $R^1$ und $R^2$ bzw. $X^1$ und $X^2$ gleich.

Insbesondere bevorzugt sind Verbindungen, die als $X^1$ und/oder $X^2$ die Gruppen $-SO_3^-$, $-OSO_3^-$, -COO⁻, $-PO_3^{2-}$, $-OP(O)(O^-)O-$ oder $-OPO_3^{2-}$, Polyethylen- oder Polypropylenglykole, $-CH(OH)-CH_2-NH-Sach$, $-Y-(CH_2-CH_2-O)_v-R^4$, Betaine, oder Sulfobetaine enthalten. Bevorzugte Gegenionen sind hierbei $H^+$, $Na^+$, $K^+$ und $NH_4^+$, insbesondere $Na^+$. Insbesondere bevorzugt sind: $-SO_3^-$, $-COO^-$, $-OP(O)(O^-)O-$ oder $-OPO_3^{2-}$, Polyethylen- oder Polypropylenglykole, Sulfobetaine, die Gruppe $-CH(OH)-CH_2-NH-Sach$ und die Gruppe $-Y-(CH_2-CH_2-O)_v-R^4$. Hierbei ist Sach = verschiedene Zucker und Y = S, O oder NH, bevorzugt O, $R^4$ = H oder Alkyl, bevorzugt H oder $CH_3$, und v = 1-100, bevorzugt 1-30, insbesondere auch 1-25. Besonders vorteilhaft können auch Verbindungen mit X = $-SO_3^-$ sein. Verbindungen der Formeln (IIa-c) und (V), vor allem solche mit den bevorzugten Variablen, sind insbesondere bevorzugt.

In einer anderen Variante der Erfindung basieren die fluorierten Verbindungen bevorzugt auf Estern der Maleinsäure und der Aconitsäure. Diese Verbindungen werden durch die Formeln (VI) und (VII) dargestellt, wobei $L^1$, $L^2$ und $L^3$ unabhängig voneinander eine gesättigte oder ungesättigte, verzweigte oder unverzweigte, ggf. Heteroatome enthaltende, Kohlenwasserstoffeinheit sind, wobei keine -O-O-Bindungen vorliegen, insbesondere eine lineare oder verzweigte C1-C6-Alkylgruppe, besonders bevorzugt eine C1-C4-Alkylgruppe, X eine hydrophile Gruppe ist und $R^1$, $R^2$ und $R^3$ unabhängig voneinander eine fluorierte, ggf. Heteroatome enthaltende, lineare oder verzweigte, Alkylgruppe sind:

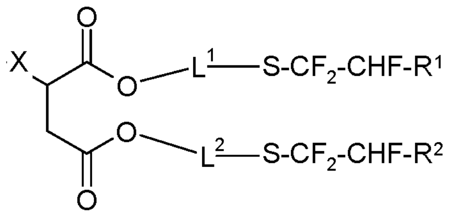

(VI)

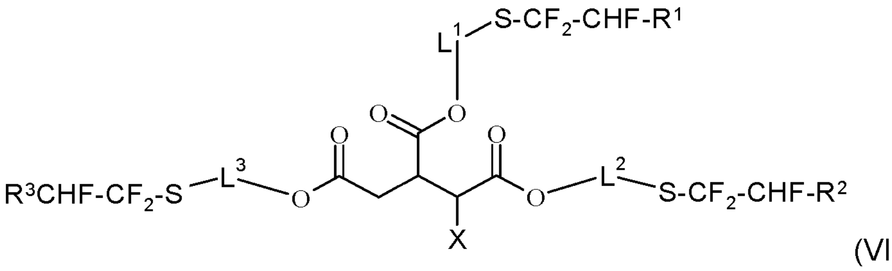

(VII)

In einer bevorzugten Variante der Verbindungen der Formeln (VI) und (VII) sind $L^1$, $L^2$ und $L^3$ unabhängig voneinander eine lineare oder verzweigte C1-C6-Alkylgruppe, besonders bevorzugt eine C1-C4-Alkylgruppe, X eine anionische oder nicht-ionische Gruppe und $R^1$, $R^2$ und $R^3$ unabhängig voneinander eine $CF_3$-$(CF_2)_{1-2}$-O- Gruppe. Bevorzugt sind $L^1$, $L^2$ und $L^3$ gleich und $R^1$, $R^2$ und $R^3$ gleich.

Besonders vorteilhaft sind Verbindungen der Formeln (I) bis (VII), in denen eine oder mehrere der Variablen die bevorzugten Bedeutungen haben. Besonders vorteilhaft sind Verbindungen der Formeln (I) bis (VII), in denen alle genannten Variablen die bevorzugten Bedeutungen, insbesondere die besonders bevorzugten Bedeutungen, haben.

Insbesondere bevorzugt sind Verbindungen der Formeln (XIII) bis (XVIII):

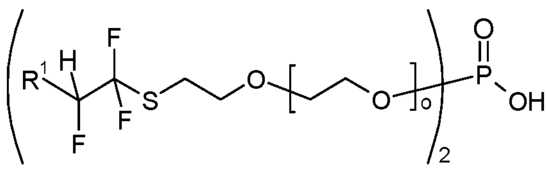

(XIII)

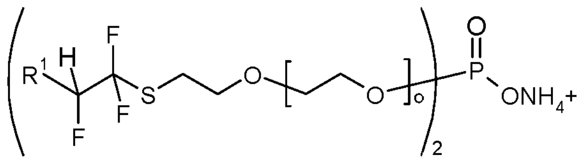

(XIII‘)

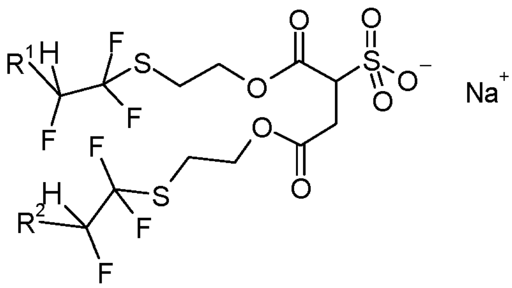

(XIV)

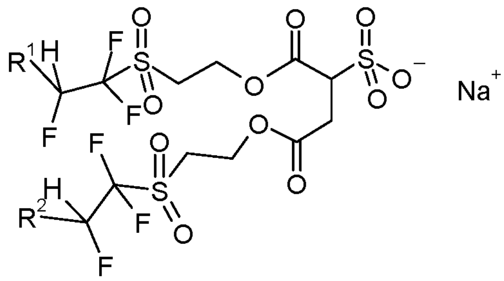

(XIVa)

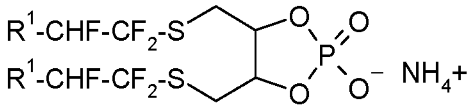

(XV)

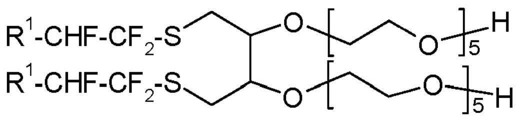

(XVI)

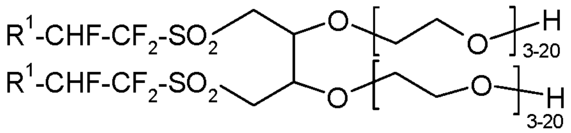

(XVIa)

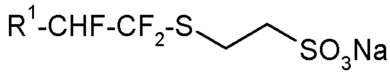

(XVII)

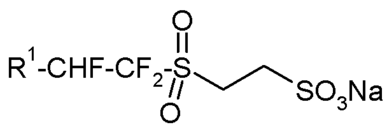

(XVIIa)

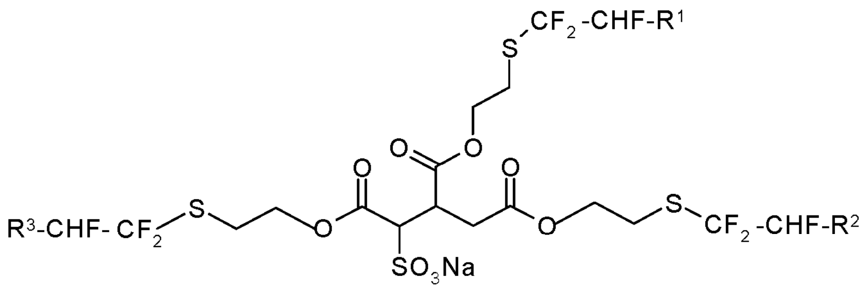

(XVIII)

in denen die fluorierten Gruppen $R^1$ und $R^2$ bzw. $R^1$, $R^2$ und $R^3$ unabhängig voneinander ausgewählt sind aus den Gruppen:

$CF_3$-$(CF_2)_{0-3}$-, $CF_3$-$(CF_2)_{0-3}$-O-, $CF_3$-$(CF_2)_{0-3}$-O-$(CF_2)_{1-3}$-,
$CF_3$-$(CF_2)_{0-3}$-O-$(CF_2)_{1-3}$-O-, $CF_3$-$(CF_2)_{0-3}$-O-$(CF_2)_{1-3}$-O-$CF_2$-,
$CF_3$-$(CF_2)_{0-3}$O-$(CF_2$-$O)_{1-8}$- und $CF_3$-$(CF_2)_{0-3}$-O-$(CF_2$-$O)_{1-8}$-$CF_2$-.

Insbesondere bevorzugt sind die fluorierten Gruppen $R^1$ und $R^2$ bzw. $R^1$, $R^2$ und $R^3$ unabhängig voneinander eine $CF_3$-$(CF_2)_{1-2}$-O- Gruppe, insbesondere eine $CF_3$-$CF_2$-$CF_2$-O-Gruppe. Besonders bevorzugt sind $R^1$ und $R^2$ gleich bzw. $R^1$, $R^2$ und $R^3$ gleich. o ist gleich 1-30, insbesondere 3, 5, 6, 10, 12, 15, 18, 20 oder 24, insbesondere 3, 10 oder 18. Verbindungen der Formeln (XIV) und (XV), (XVI), (XVII) und (XVIII), vor allem solche mit den bevorzugten Variablen, sind insbesondere bevorzugt.

Insbesondere die folgenden Verbindungen der Formeln (XIX) bis (XXIV), mit o = 0, 10 oder 18 bzw. R = Methyl oder Ethyl, sind besonders bevorzugt:

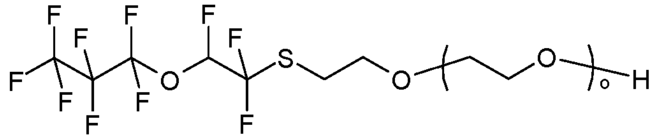

(XIXa)

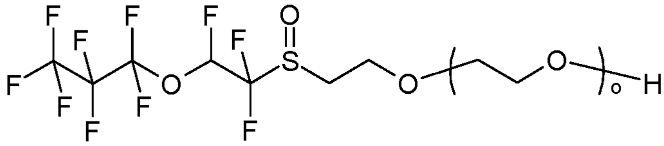

(XIXb)

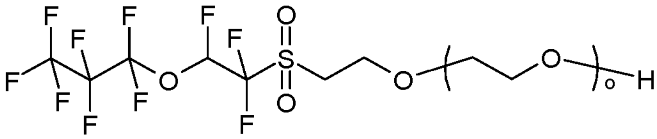

(XIXc)

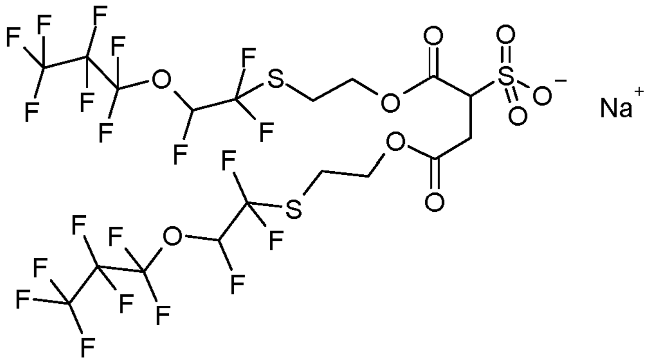

(XX)

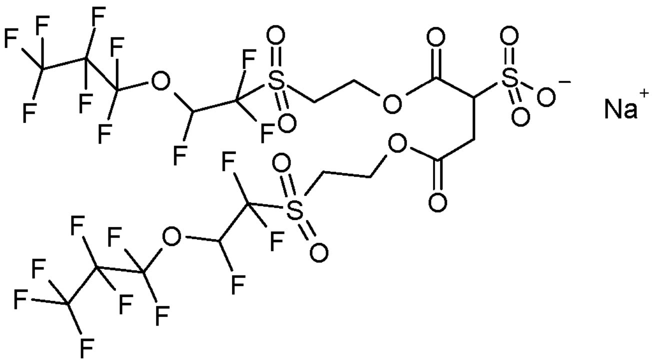

(XXa)

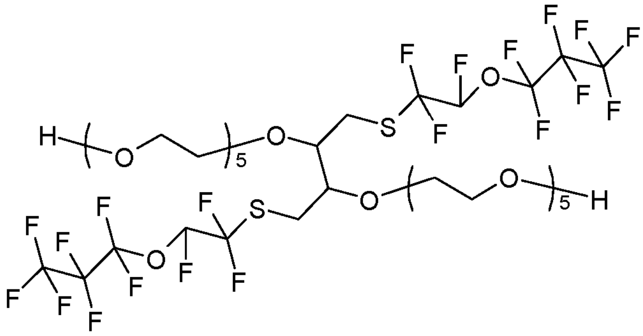

(XXI)

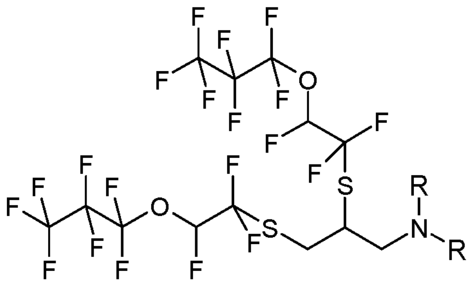
(XXII)

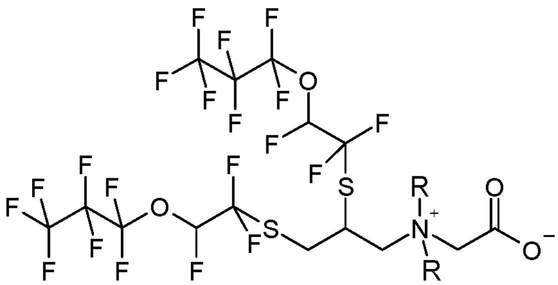
(XXIII)

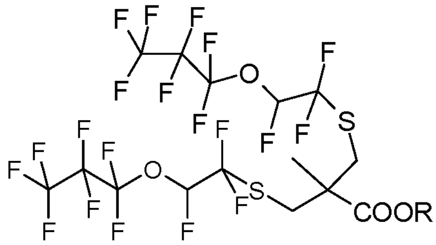
(XXIV)

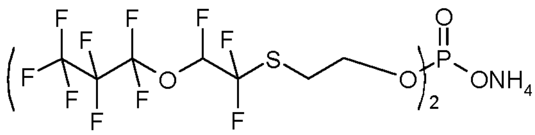
(XXV)

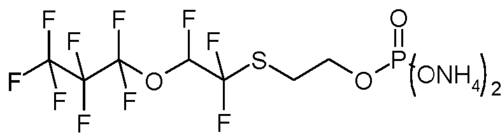
(XXVI)

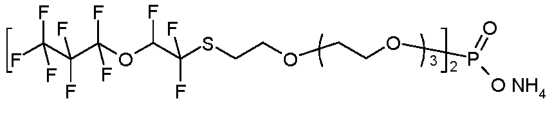
(XXVII)

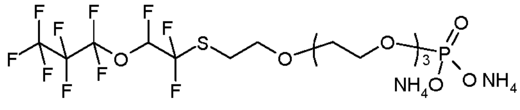
(XXVIII)

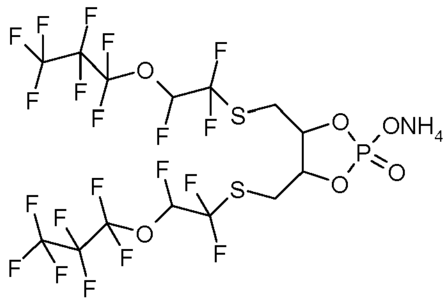

(XXIX)

Verbindungen der Formeln (XIXa), (XX) bis (XXIX), vor allem solche mit den bevorzugten Variablen, sind insbesondere bevorzugt.

Die erfindungsgemäßen Fluortenside auf Basis von Perfluorolefinen besitzen einen geringere Stabilität als herkömmliche Fluortenside und können daher leichter durch physikalisch/ chemische Prozessen abgebaut werden, und sie sind bevorzugt nicht persistent. Die erfindungsgemäßen Fluortenside können sich durch eine sehr effiziente Erniedrigung der Oberflächenspannungsenergie in wässrige Lösungen auszeichnen. Darüber hinaus besitzen diese Verbindungen bevorzugt einen niedrige CMC sowie ein geringes Schaumverhalten.

Ferner ermöglicht die Einführung der Sulfidbrücke eine Variantionserweiterung der Molekülstruktur. Sulfide lassen sich bekanntlich mit für den Fachmann aus der Literatur bekannten Methoden in Sulfoxide überführen, was ein zusätzliches "Trimmen" der Molekülpolarität in Hinblick auf Hydrophilie erlaubt.

Die erfindungsgemäßen Verbindungen sind nach dem Fachmann bekannten Verfahren herstellbar. Mit Hilfe von Perfluorolefinen und heterofunktionelle Molekülen, lassen sich gezielt die neuen Fluortenside herstellen, die mehrere positive Effekte in sich vereinen können. Es hat sich gezeigt, dass Thiol Verbindungen aufgrund Ihrer erhöhten Nucleophilie eine deutlich höhere Reaktivität aufweisen als vergleichbare Alkohole. Diesen Vorteil kann man sich nun zu Nutzen machen, indem man mono- oder polyfunktionelle Alkohole, die zusätzlich noch über ein oder mehrere Thiolgruppen verfügen, selektiv und ohne Einführung einer Schutzgruppe an der Schwefelgruppe verethert. Die freien OH-Gruppen können dann in einem zweiten Schritt weiter umgesetzt werden. Diese Eigenschaft führt dazu, dass in Vergleich zu den entsprechenden Alkoholverbindungen die Syntheseführung stark vereinfacht werden kann und die Ausbeute erhöht ist.

Die folgenden Schemata 1 bis 6 zeigen Beispielsynthesen für erfindungsgemäße Verbindungen. Diese Verfahren sind dem Fachmann generell bekannt und können unter üblichen Bedingungen durchgeführt werden. Bevorzugt können die folgenden beispielhaft genannten Perfluorolefinverbindungen eingesetzt werden:

$CF_3$-$CF$=$CF_2$ $CF_3$-$CF_2$-$CF_2$-$OCF$=$CF_2$

$CF_3$-$CF_2$-$CF$=$CF_2$ $CF_3$-$CF_2$-$OCF$=$CF_2$

$CF_3$-$CF_2$-$CF_2$-$CF$=$CF_2$ $CF_3$-$OCF$=$CF_2$

Die erfindungsgemäßen Verbindungen, insbesondere Verbindungen der Formeln (II) bis (VII) können bevorzugt nach den folgenden Syntheserouten (beispielhaft dargestellt für Verbindungen mit $R^1$ = $CF_3CF_2CF_2O$-) hergestellt werden.

Schema 1:

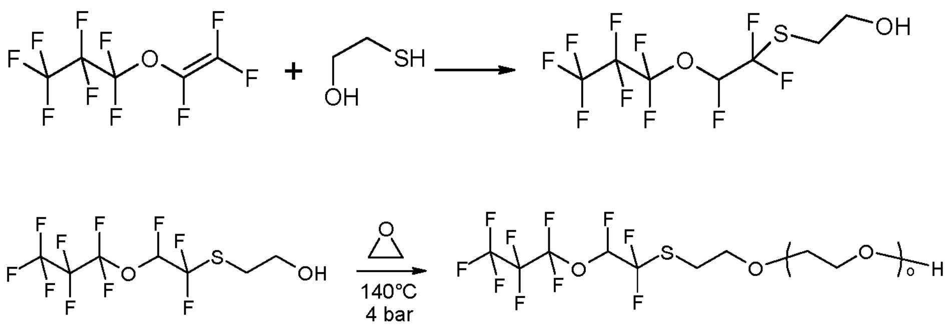

Schema 1a:

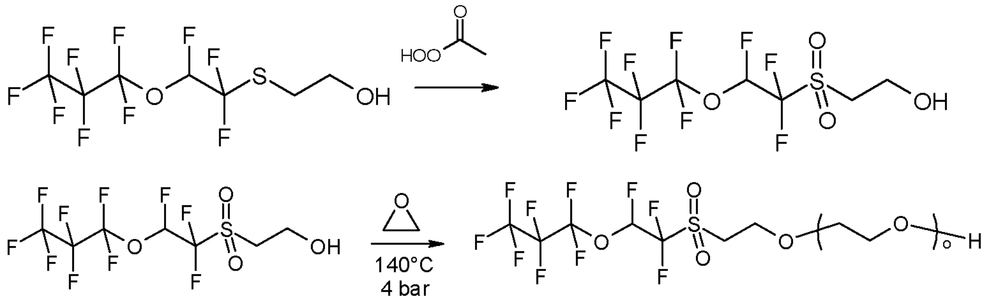


Schema 2:

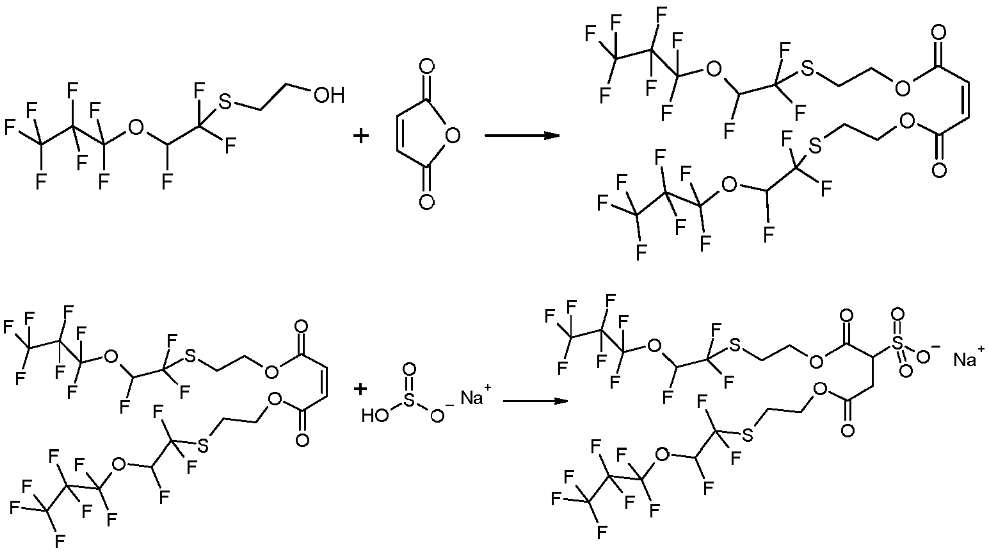

Schema 2a:

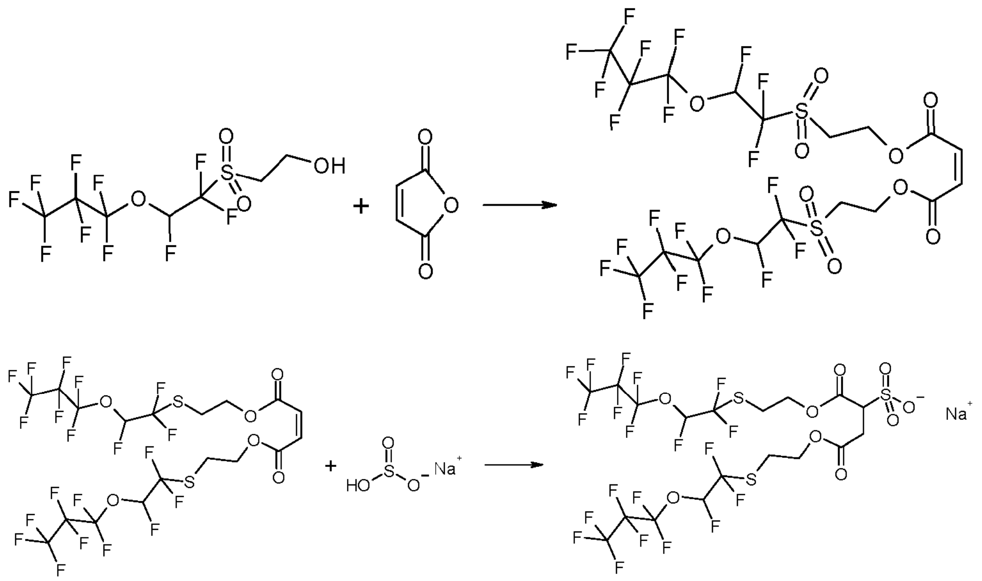

Schema 3:

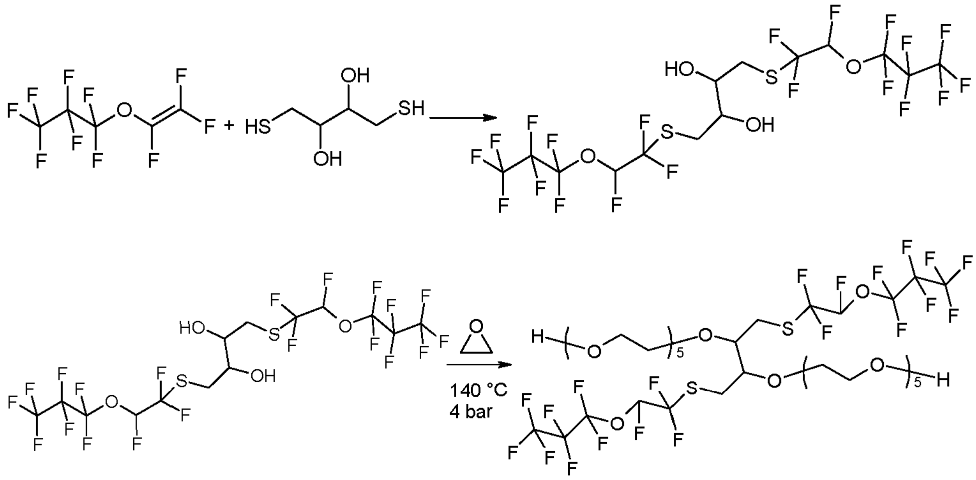


Schema 4:

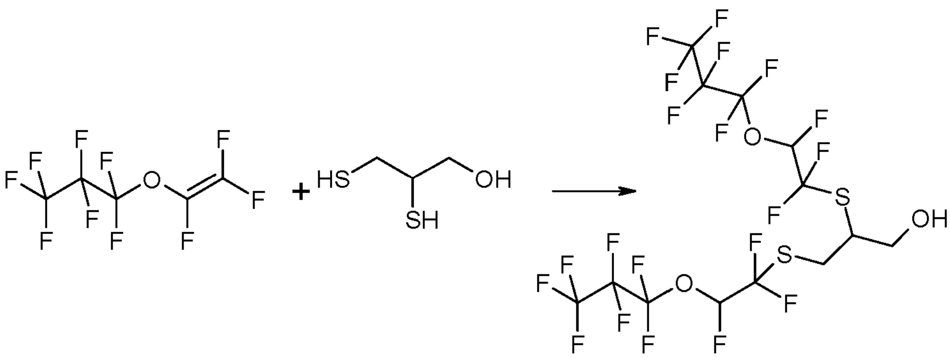

Schema 5:

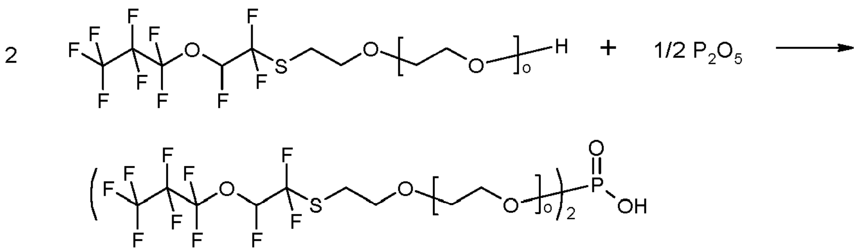

Schema 5a:

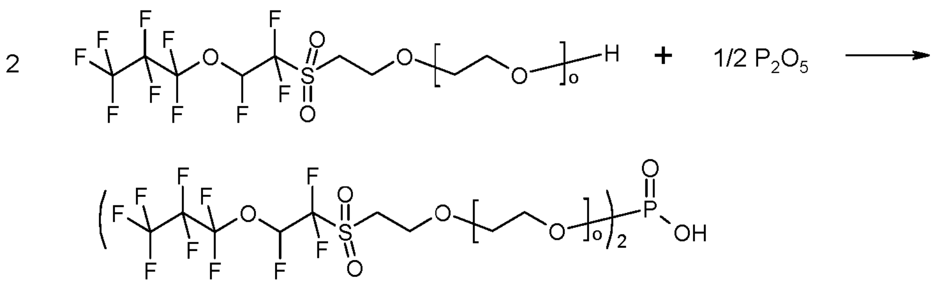

Schema 6:

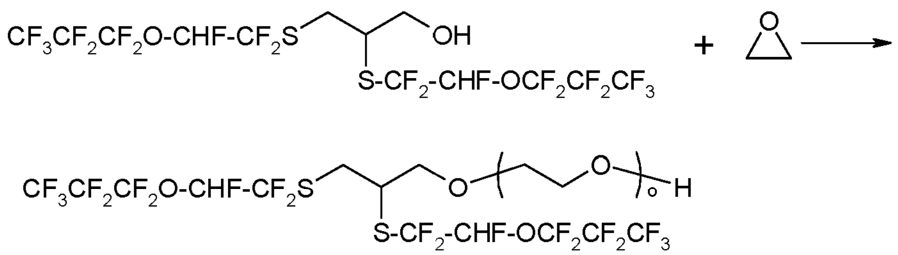

Die Herstellung weiterer erfindungsgemäßer Verbindungen kann analog zu den oben gezeigten beispielhaften Reaktionen oder nach anderen dem Fachmann an sich aus der Literatur bekannten Methoden erfolgen. Besonders bevorzugt sind hierbei Verbindungen der Formeln (XIII) bis (XXIV). Diese Verfahren sind dem Fachmann generell bekannt und können unter üblichen Bedingungen durchgeführt werden. Die verwendeten Ausgangsverbindungen sind kommerziell erhältlich und/oder ihre Herstellung ist dem Fachmann geläufig.

Vorteile der erfindungsgemäßen Verbindungen können insbesondere sein:

- eine Oberflächenaktivität, die der konventioneller Kohlenwasserstoff-Tenside hinsichtlich Effizienz und/oder Effektivität gleich oder überlegen ist,
- biologische und/oder abiotische Abbaubarkeit der Substanzen ohne Bildung persistenter perfluorierter Abbauprodukte wie PFOA (Perfluoroctansäure) oder PFOS (Perfluoroctansulfonat),
- nach einfachen Verfahren herstellbar,
- schwache Schaumwirkung und/oder geringe Schaumstabilisierung,
- gute Verarbeitbarkeit in Formulierungen und/oder
- Lagerstabilität.

Bevorzugt können die erfindungsgemäßen Verbindungen eine besondere Oberflächenaktivität aufweisen. Die erfindungsgemäßen Verbindungen der Formel (I), insbesondere die Verbindungen der Formeln (IIa) bis (VII) und bevorzugt der Formeln (XIII) bis (XXIV) können außerdem verbesserte Umwelteigenschaften aufweisen, da sie weder chemisch noch biologisch zu langkettigen PFCAs oder PFASs abbauen. Bevorzugt können die erfindungsgemäßen Verbindungen durch entsprechende Umwelteinflüsse vollständig in mineralisierbare/regenerierbare Verbindungen überführt werden.

Ein Verfahren zum Abbau der fluorhaltigen Verbindungen umfasst die folgenden Schritte:

a) biologische und/oder abiotischer Abbau des Kohlenstoffgerüsts der fluorhaltigen Verbindungen unter Bildung von, vorzugsweise nicht toxischen, fluorhaltigen Verbindungen mit einem ausreichend hohen Dampfdruck,
b) Überführen der in Schritt a) gebildeten fluorhaltigen Verbindungen mit hohem Dampfdruck in eine Gasphase,
c) Abbau der in Schritt a) gebildeten fluorhaltigen Verbindungen mit hohem Dampfdruck zu niedermolekularen Verbindungen durch UV-Bestrahlung in der Gasphase,
d) Überführen der in Schritt c) gebildeten niedermolekularen Verbindungen aus der Gasphase in eine flüssige und/oder feste Phase,
e) Mineralisierung der in Schritt c) gebildeten niedermolekularen Verbindungen der flüssigen und/oder festen Phase.

Bevorzugt werden in Schritt a) keine fluorhaltigen, Salze gebildet. Insbesondere werden in Schritt a) keine perfluorierten Verbindungen gebildet.

Die Verbindungen der Formeln (I) bis (XXIV) können bevorzugt als oberflächenaktive Mittel verwendet werden, bevorzugt als Tensid, Hydrophobiermittel, Grenzflächenvermittler, Viskositätsminderer, Schaumstabilisator oder Emulgator.

Ein weiterer Gegenstand der vorliegenden Erfindung ist die Verwendung der erfindungsgemäßen Verbindungen und den vorstehend beschriebenen bevorzugten Ausführungsformen als oberflächenaktive Mittel, beispielsweise zur Verbesserung des Verlaufsverhaltens und des Benetzungsvermögens von Coatingformulierungen. Fluortenside der Formeln (I) bis (VII), insbesondere der Formeln (XIII) bis (XXIV) werden bevorzugt verwendet, insbesondere die genannten besonders bevorzugten Verbindungen

Neben den Verbindungen der Formel (I), insbesondere der bevorzugten Verbindungen der Formeln (II) bis (VII), vor allem der Formeln (XIII) bis (XXIV), können die erfindungsgemäßen Mischungen auch Lösemittel, Additive, Hilfs- und Füllstoffe sowie nicht fluorierte Tenside enthalten.

Beispielhaft seien genannt Silikonpartikel, Weichmacher und oberflächenmodifizierte Pigmente.

Bevorzugte Einsatzgebiete sind beispielsweise die Verwendung der erfindungsgemäßen Fluortenside der Formel (I) und der bevorzugten Verbindungen als Additive in Zubereitungen zur Oberflächenbeschichtung, wie Farben, Lacken, Schutzanstrichen, Spezialcoatings in elektronischen oder Halbleiter-Anwendungen (z.B. Photolacken, Top Antireflective Coatings, Bottom Antireflective Coatings) oder in optischen Anwendungen (z.B. photographischen Beschichtungen, Beschichtungen optischer Elemente), in Agrochemikalien, in Poliermitteln und Wachsen, z.B. für Mobiliar, Fußböden und Automobile, insbesondere in Bodenpolituren, in Feuerlöschmitteln, Schmierstoffen, in photolithographischen Verfahren, insbesondere in Immersionsphotolithographie-Verfahren, z.B. in Entwicklerlösungen, Spüllösungen, Immersionsölen und/oder in den Photoresists selbst, vor allem zur Herstellung von gedruckten Schaltungen oder in Additivzubereitungen zur Additivierung entsprechender Zubereitungen. Darüber hinaus eignen sich die erfindungsgemäß als Tensid verwendbaren Verbindungen für Wasch- und Reinigungsanwendungen, sowie für eine Verwendung als Additive/Tenside in kosmetischen Produkten wie z. B. Haar- und Körperpflegeprodukten (z.B. Shampoos, Haarspülungen und Haarkonditionierern), Schaumbädern, Cremes oder Lotionen mit einer oder mehreren der folgenden Funktionen: Emulgatoren, Netzmittel, Schaummittel, Gleitmittel, Antistatikum, Erhöher der Resistenz gegen Hautfette.

Dabei werden die erfindungsgemäßen Fluortenside für die Anwendung üblicherweise in entsprechend ausgelegten Zubereitungen eingebracht. Übliche Einsatzkonzentrationen sind 0.01 - 1.0 Gew.-% der erfindungsgemäßen Tenside bezogen auf die Gesamtzubereitung.

Entsprechende Mittel, enthaltend die erfindungsgemäßen Fluortensiden, sind ebenfalls Gegenstand der vorliegenden Erfindung. Bevorzugt enthalten solche Mittel einen für den jeweiligen Verwendungszweck geeigneten Träger, sowie gegebenenfalls weitere Aktivstoffe und/oder gegebenenfalls Hilfsstoffe. Bevorzugte Mittel sind Farb- und Lackzubereitungen, Feuerlöschmittel, Schmierstoffe, Wasch- und Reinigungsmittel und Enteiser oder Entwicklerlösungen, Spüllösungen, Immersionsöle und Photoresists für photolithograqphische Verfahren, insbesondere für Immersionsphotolithographie-Verfahren und insbesondere zur Herstellung gedruckter Schaltungen, Agrochemikalien, Bodenpolituren, kosmetische Produkte oder Hydrophobiermittel zur Textilausrüstung oder Glasbehandlung. Bei bevorzugten Mitteln handelt es sich dabei um Farb- und Lackzubereitungen und Druckfarben.

Außerdem sind auch wasserbasierte Lackformulierungen, die die erfindungsgemäßen Fluortenside allein oder im Gemische mit Additiven enthalten, Gegenstand der vorliegenden Erfindung. Bevorzugt werden Lackformulierungen auf Basis der folgenden synthetischen Filmbildner verwendet: Polykondensationsharze wie Alkydharze, gesättigt/ungesättigte Polyester, Polyamide/imide, Silikonharze; Phenolharze; Harnstoffharze und Melaminharze, Polyadditionsharze wie Polyurethane und Epoxidharze, Polymerisationsharze wie Polyolefine, Polyvinylverbindungen und Polyacrylate.

Außerdem sind die erfindungsgemäßen Fluortenside auch zum Einsatz in Lacken auf Basis von Naturstoffen und modifizierten Naturstoffen geeignet. Bevorzugt sind Lacke auf Basis von Ölen, Polysacchariden wie Stärke und Cellulose als auch auf Basis von Naturharzen wie cyclischen Oligoterpenen, Polyterpenen und/oder Schellack.

Die erfindungsgemäßen Fluortenside können sowohl in physikalisch härtenden (Thermoplaste) als auch in vernetzenden (Elastomere und Duromere) wässrigen Lacksystemen verwendet werden. Bevorzugt verbessern die erfindungsgemäßen Fluortenside die Verlaufs- und Benetzungseigenschaften der Lacksysteme.

Alle hier genannten Verwendungen erfindungsgemäß einzusetzender Fluortenside, insbesondere der bevorzugten Verbindungen, sind Gegenstand der vorliegenden Erfindung. Die jeweilige Anwendung von Fluortenside zu den genannten Zwecken ist dem Fachmann bekannt, so dass der Einsatz der erfindungsgemäß einzusetzenden Fluortenside keine Probleme bereitet.

Die folgenden Beispiele erläutern die vorliegende Erfindung näher, ohne den Schutzbereich zu beschränken.

## Beispiele

Die NMR-Spektren werden mit einem Bruker 400 MHz Spektrometer mit internem Standard gemessen.

Die IR-Spektren werden mit einem Brucker Alpha Platinum-ATR Spektrometer gemessen.

## Bestimmung der statischen Oberflächenspannung

Es werden die statischen Oberflächenspannungen $\gamma$ von wässrigen Tensidlösungen mit verschiedenen Konzentrationen c (Gramm pro Liter) bestimmt.

Gerät: Tensiometer der Firma Dataphysics (Modell DCAT 11)
Temperatur der Messlösungen: 20°±0,2°C
Eingesetzte Messmethode: Messung der Oberflächenspannung mit der Wilhelmy Plattenmethode nach DIN EN 14370.
Platte: Platin, Länge= 19,9mm

Bei der Plattenmethode wird die Oberflächen- bzw. Grenzflächenspannung der Tensidlösung aus der auf die benetzte Länge einer Platte wirkenden Kraft nach folgender Formel berechnet: $\gamma = \frac{F}{L \cdot \cos\theta} = \frac{F}{L}$ $\gamma$= Grenz- oder Oberflächenspannung; F= auf die Waage wirkende Kraft; L= benetzte Länge (19,9 mm); $\theta$= Kontaktwinkel)Die Platte besteht aus angerautem Platin und wird also optimal benetzt, so dass der Kontaktwinkel $\theta$ nahe bei 0° liegt. Der Term cos $\theta$ erreicht daher annähernd den Wert 1, so dass nur noch die gemessene Kraft und die Länge der Platte berücksichtigt werden müssen.

**Abkürzungen**

| | |
|---|---|
| EO | Ethylenoxideinheiten |
| THF | Tetrahydrofuran |
| MTBE | tert-Butylmethylether |
| Sdp. | Siedepunkt |
| w% | Gewichtsprozent |

Beispiel 1: Synthese von Verbindungen der Formel (XIX)

Beispiel 1a:

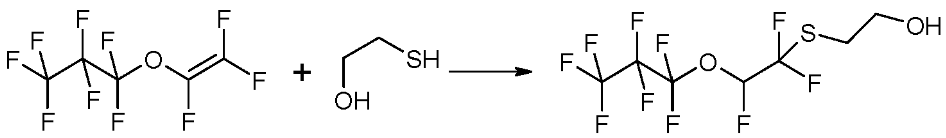

In einem Druckreaktor werden 77,3g 1,1,1,2,2,3,3-Heptafluoro-3-trifluorovinyloxy-propan, 52,2g 2-Mercaptoethanol, 12,05g Kaliumcarbonat und 50mL Acetonitril zusammengegeben. Das Reaktionsgemisch wird bei 100°C 20h gerührt. Das Reaktionsgemisch wird mit jeweils 50mL Wasser und MTBE versetzt und die Phasen getrennt. Die wässrige Phase wird mit 2x30mL MTBE extrahiert. Anschließend wird die vereinigte organische Phase mit 40mL Wasser und 40mL gesättigter NaCl-Lösung gewaschen. Das Extrakt wird über Natriumsulfat getrocknet und das Lösemittel abdestilliert. Der Rückstand wird danach im Vakuum fraktioniert destilliert. Das Produkt weist dabei eine Kopftemperatur von 49°C bei einem Druck von 0,058mbar auf. Auswaage: 63,47g

1H-NMR: 7,2 ppm (dt, 1 H, -CFH); 5,1ppm (t, 1H, =OH);
3,6 ppm (q, 2H, -CH2-OH ); 3,0 ppm (t,2H, S-CH2- CH2)

**Beispiel 1b:**

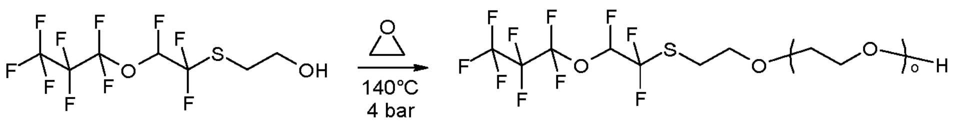


In einem Druckreaktor wird der in Beispiel 1a hergestellte Alkohol mit Ethylenoxid bei 140°C und max 4 bar zu dem entsprechenden Fluortensid umgesetzte. Entsprechend der Reaktionszeit können unterschiedliche Kettenlängen realisiert werden. Die hier synthetisierten Materialien haben statistische EO Kettenlängen von 3, 10 bzw. 18 Einheiten.

**Beispiel 1c:** n=3

1H-NMR: 7,2 ppm (dt, 1 H, -CFH); 3,6 ppm (q, 2H, -CH2-OH); 3,55-3,40(m, 12H, -CH2CH2-O); 3,0 ppm (t,2H, S-CH2- CH2)

**Beispiel 1d:** n=10

1H-NMR: 7,2 ppm (dt, 1 H, -CFH); 3,6 ppm (q, 2H, -CH2-OH); 3,55-3,40(m, 42H, -CH2CH2-O); 3,0 ppm (t,2H, S-CH2- CH2)

**Beispiel 1e:** n=18

1H-NMR: 7,2 ppm (dt, 1 H, -CFH); 3,6 ppm (q, 2H, -CH2-OH); 3,55-3,40(m, 74H, -CH2CH2-O); 3,0 ppm (t,2H, S-CH2- CH2)

**Beispiel 1f:**

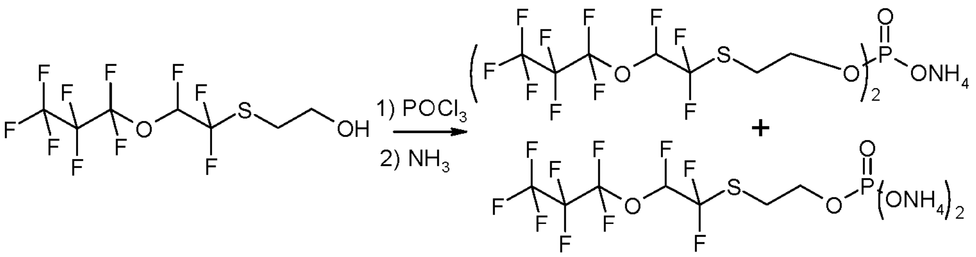


In einem Reaktionskolben werden 11 g des Alkohols aus Beispiel 1a in 4 ml Toluol vorgelegt und auf 60°C erhitzt. Innerhalb von 10min werden langsam 2,00 g Phosphorylchlorid zugegeben. Anschließend wird das Reaktionsgemisch auf 115°C erhitzt und 5,5h bei dieser Temperatur gerührt. Anschließend wird der Ansatz auf 90°C abgekühlt, vorsichtig mit 0,3 mL Wasser hydrolysiert und das Gemisch eine weitere Stunde bei dieser Temperatur gerührt. Anschließend wird das Lösungsmittel entfernt und ein brauner Rückstand verbleibt.

Das Rohprodukt wird mit 20 ml MTBE und 20 Wasser versetzt und die Phasen getrennt. Die wässrige Phase wird mit 2x30mL MTBE extrahiert. Anschließend wird die vereinigte organische Phase mit Ammoniaklösung neutralisiert und abgetrennt.
Auswaage: 11,18g.

1H-NMR: 7,3-7,1 ppm (m, 2 H, -CFH); 4,0 -3,2 ppm (m, S-CH2-CH2);
31 P-NMR: -0.7ppm (t, 2 P); -1,8 ppm (quin, 1P) d.h. Mono- und Diester liegen im Verhältnis 2/1 vor.

**Beispiel 1 g:**

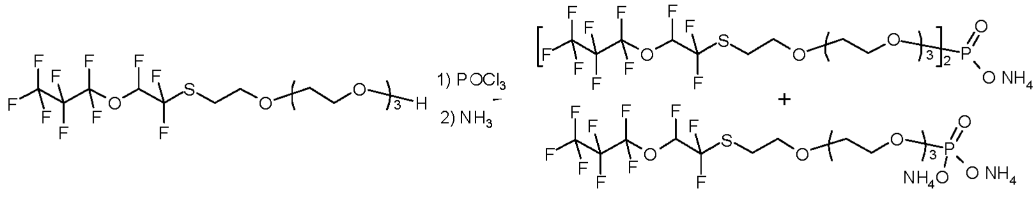


11 g des Alkohols von Beispiel 1c werden analog zu Beispiel 1f mit 1,5 g $POCl_3$ umgesetzt.
Ausbeute 11,0g
1H-NMR: 7,3-7,1 ppm (m, 2 H, -CFH); 3,8-3,3 ppm (m, 4H, S-CH2-CH2) und (m,24H,CH2-CH2-O);

31 P-NMR: -0.7ppm (t, 3 P); -1,8 ppm (quin, 2 P) d.h. Mono- und Diester liegen im Verhältnis 2/1 vor.

**Beispiel 1h:**

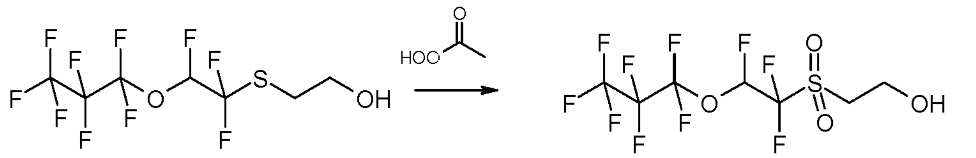


In einem Rundkolben werden 6,5 g des in Beispiel 1a hergestellten Alkohol in 24ml Acetonitril vorgelegt und langsam 5,4 ml 40%ig Peressigsäure unter Rühren zugetropft. Das Reaktionsgemisch wird dann auf 80°C erhitzt und für 24h bei dieser Temperatur gerührt. Das Reaktionsgemisch wird mit 30 ml Wasser und 30ml MTBE versetzt und die Phasen getrennt. Die wässrige Phase wird mit 2x30mL MTBE extrahiert. Anschließend wird die vereinigte organische Phase mit jeweils 40mL Wasser und 40mL gesättigter NaCl-Lösung gewaschen. Das Extrakt wird über Natriumsulfat getrocknet und das Lösemittel abdestilliert. Auswaage: 6,69g

1H-NMR: 8,2 und 7,2 ppm (m, 1 H, -CFH);
4,0 ppm (m, 4H, SO2-CH2-CH2-OH )

**Beispiel 2:** Synthese der Verbindung der Formel (XX)

**Beispiel 2a:** Herstellung des fluorierten Maleinsäureesters

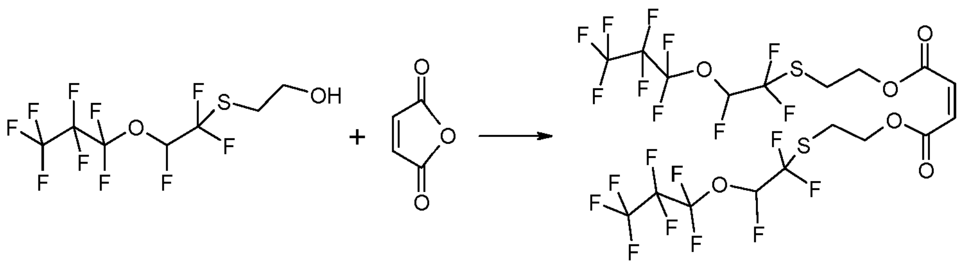

In einem Rundkolben werden 10,49g des unter Beispiel 1a hergestellten Alkohol, 1,30g Maleinsäureanhydrid, 0,68g p-Toluolsulfonsäure-Monohydrat in 30mL Toluol zusammengegeben. Das Reaktionsgemisch wird für 24h am Wasserabscheider unter Rückfluss gerührt. Das Reaktionsgemisch wird mit jeweils 30mL Wasser und MTBE versetzt und die Phasen getrennt. Die wässrige Phase wird zweimal mit 20mL MTBE extrahiert. Anschließend wird die vereinigte organische Phase mit 30mL Wasser und 30mL gesättigter NaCl-Lösung gewaschen. Das Extrakt wird über Natriumsulfat getrocknet und das Lösemittel abdestilliert. Auswaage: 10,68g

1H-NMR: 7,2ppm (dt, 2 H, -CFH); 6,5ppm (m, 2H, -CH=CH-);
4,3 ppm (t, 2H, -CH2-CH2-O); 3,2 ppm (t, 2H, -CH2-CH2-S-)

**Beispiel 2b:** Herstellung des fluorierten Sulfosuccinates

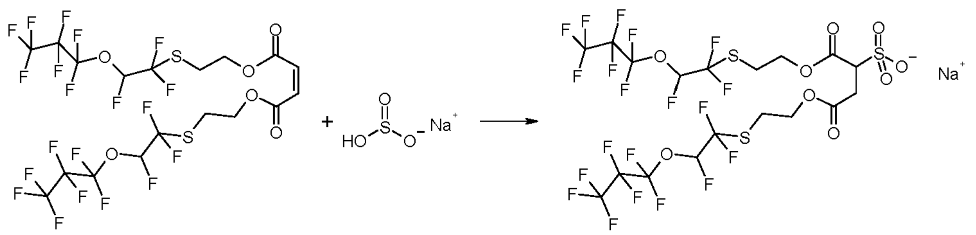

In einem Rundkolben werden 10,00g des fluorhaltigen Maleinsäureesters, 2,71g einer 39%-igen Natriumhydrogensulfitlösung und 30mL 2-Propanol vorgelegt. Danach wird der pH-Wert des Reaktionsgemischs mit Natriumhydroxidlösung auf pH 6,3 eingestellt. Anschließend wird das Reaktionsgemisch für 96h bei 95°C gerührt. Das Reaktionsgemisch wird mit 30mL MTBE und 30mL Wasser versetzt und die Phasen getrennt. Die wässrige Phase wird zweimal mit 20mL MTBE extrahiert. Anschließend wird die vereinigte organische Phase mit 30mL Wasser und 30mL gesättigter NaCl-Lösung gewaschen. Das Extrakt wird über Natriumsulfat getrocknet und das Lösemittel abdestilliert. Ausbeute: 6,64g

1H-NMR: 7,2 ppm (dt, 2 H, -CFH); 4,2 ppm (m, 4H, S-CH2-CH2);
3,7 ppm (dd, 1H, -CH-SO3- ); 3,2 ppm (t,4H,CH2-CH2-O);
2,8-3,0 ppm (m, 2 H, -CH2-CH);

<u>**Beispiel 2c:** Vorstufe zur Synthese der Verbindung der Formel (XVIII)</u>

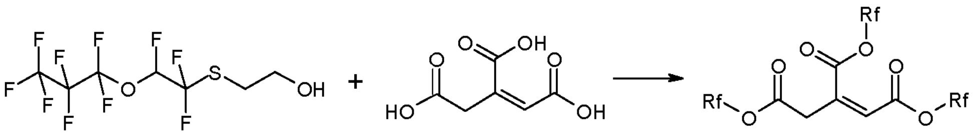

In einem Rundkolben werden 9,49 g des unter Beispiel 1a hergestellten Alkohol, 1,50g Aconitsäure, 0,45g p-Toluolsulfonsäure-Monohydrat in 50 mL Toluol zusammengegeben. Das Reaktionsgemisch wird für 72h am Wasserabscheider bei 115°C gerührt. Das Reaktionsgemisch wird mit jeweils 30mL Wasser und MTBE versetzt und die Phasen getrennt. Die wässrige Phase wird zweimal mit 20mL MTBE extrahiert. Anschließend wird die vereinigte organische Phase mit 30mL Wasser und 30mL gesättigter NaCl-Lösung gewaschen. Das Extrakt wird über Natriumsulfat getrocknet und das Lösemittel entfernt. Auswaage: 6,78g 1H-NMR: 7,8-7,6 ppm (m, 2 H, -CFH); 6,8 ppm (s, H, -C=C**H**-); 4,4 - 4,0 ppm (t, 2H, -CH2-C**H**2-S-); 3,7 ppm (s, H, C**H**2--C=CH-); 3,2- 2,8 ppm (t, 2H, -C**H**2-CH2-O)

<u>**Beispiel 2d:** Synthese der Verbindung der Formel (XVIII)</u>

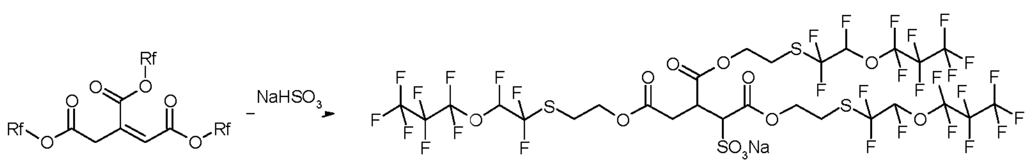


In einem Rundkolben werden 6,78g des fluorhaltigen Aconitsäureesters aus Beispiel 2c, 1,22g einer 39%-igen Natriumhydrogensulfitlösung und 24 mL 2-Propanol vorgelegt. Danach wird der pH-Wert des Reaktionsgemischs mit Natriumhydroxidlösung auf pH 6,3 eingestellt. Anschließend wird das Reaktionsgemisch für 96h bei 95°C gerührt. Das Reaktionsgemisch wird mit 30mL MTBE und 30mL Wasser versetzt und die Phasen getrennt. Die wässrige Phase wird zweimal mit 20mL MTBE extrahiert. Anschließend wird die vereinigte organische Phase mit 30mL Wasser und 30mL gesättigter NaCl-Lösung gewaschen. Das Extrakt wird über Natriumsulfat getrocknet und das Lösemittel entfernt. Ausbeute: 6,26g 1H-NMR: 7,0 ppm (dt, 3 H, -CFH); 4,2-3,8 ppm (m, 6H, S-C**H**2-CH2); 3,5-2,5 ppm (m,10H,CH2-C**H**2-O) und C**H**2-RC**H**-C**H**-SO3H

<u>**Beispiel 3:** Synthese der Verbindung der Formel (XXI) mit n=5; 7,5;10</u>

**Beispiel 3a:** Herstellung des fluorierten Diols

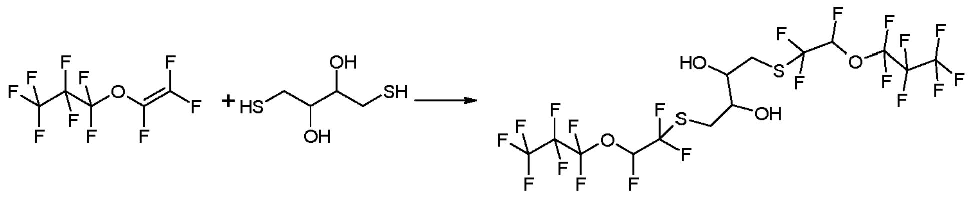

In einem Druckreaktor werden 8,63g 1,1,1,2,2,3,3-Heptafluoro-3-trifluorovinyloxy-propan, 2,50g 1,4-Dimercapto-butan-2,3-diol, 0,67g Kaliumcarbonat und 30mL Acetonitril zusammengegeben und für 20h bei 120°C gerührt. Das Reaktionsgemisch wird mit 30mL Wasser und 30mL MTBE versetzt und die Phasen getrennt. Die wässrige Phase wird zweimal mit 20mL MTBE extrahiert. Anschließend wird die vereinigte organische Phase mit 30mL Wasser und 30mL gesättigter NaCl-Lösung gewaschen. Danach wird das Extrakt über Natriumsulfat getrocknet und das Lösemittel abdestilliert. Auswaage: 9,82g

1H-NMR: 7,2 ppm (dt, 2 H, -CFH); 5,3 ppm (m, 2H, -OH);
3,7 ppm (dt, 2H, CH2-CH-O-); 2,9-3,2 ppm (m, 4 H, CH2-S);

**Beispiel 3b:** Herstellung der ethoxylierten Verbindung

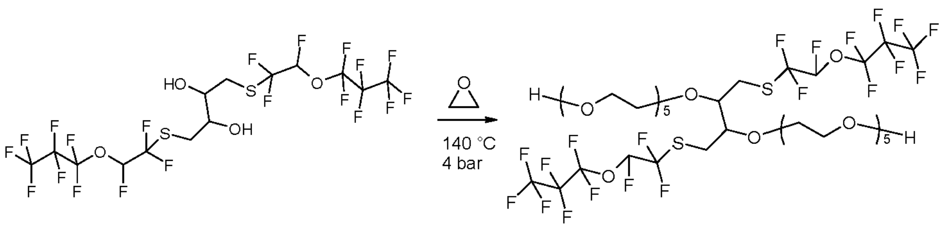


In einem Druckreaktor wird der hergestellte Alkohol mit Ethylenoxid bei 140°C und max 4 bar Druck zu dem entsprechenden Fluortensid umgesetzte. Entsprechend der Reaktionszeit können unterschiedliche Kettenlängen realisiert werden. Das hier synthetisierte Material hat eine statistische EO Kettenlänge von 5 Einheiten.
1H-NMR: 7,2 ppm (dt, 2 H, -CFH); 3,85-3,4 ppm (m, 44H); 2,9-3,2 ppm (m, 4 H, CH2-S);

**Beispiele 3 c-e:**

Erfolgt analog zum Beispiel 3b nur wird länger ethoxyliert bis eine durchschnittliche Wiederholungsanzahl von 3c: EO = 10; 3d: EO=15; 3e EO = 20 erreicht ist.

**Beispiel 3 f:**

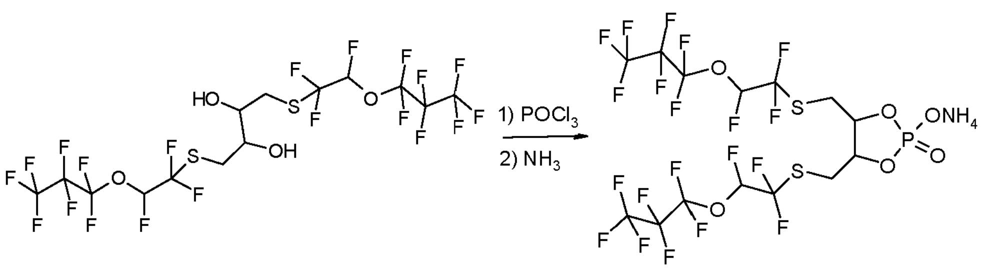


In einem Reaktionskolben werden 11 g der Verbindung aus Beispiel 3a in 4 ml Toluol vorgelegt und auf 60°C

erhitzt. Innerhalb von 10min werden langsam 1,98 g Phosphorylchlorid zugegeben. Anschließend wird das Reaktionsgemisch auf 115°C erhitzt und 18h bei dieser Temperatur gerührt. Anschließend wird der Ansatz auf 90°C abgekühlt, vorsichtig mit 0,3 mL Wasser hydrolysiert und das Gemisch eine weitere Stunde bei dieser Temperatur gerührt. Anschließend wird das Lösungsmittel entfernt und ein brauner Rückstand verbleibt.

Das Rohprodukt wird mit 20 ml MTBE und 20 ml Wasser versetzt und die Phasen getrennt. Die wässrige Phase wird mit 2x30mL MTBE extrahiert. Anschließend wird die vereinigte organische Phase mit Ammoniaklösung neutralisiert und abgetrennt.

Auswaage: 11,18g.
1H-NMR: 7,2 ppm (dt,2 H, -CFH); 3,75 ppm (m, 2H, ROC**H**-C**H**OR); 2,75 ppm (m, 4 H, S-C$\mathbf{H}_2$-CHR)

**Beispiel 4:** Synthese von Verbindungen der Formel (XVII)

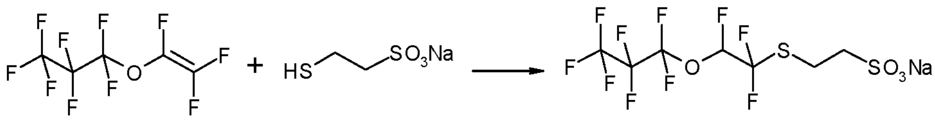

In einem Druckreaktor werden 7,53 g 1,1,1,2,2,3,3-Heptafluoro-3-trifluorovinyloxy-propan, 3,58 g des Natriumsalzes der 2-Mercaptoethansulfonsäure, 0,90g Kaliumcarbonat und 30mL Acetonitril zusammengegeben und für 18h bei 110°C gerührt Das Reaktionsgemisch wird mit MTBE und Wasser versetzt und die Phasen getrennt. Die wässrige Phase wird mit 2x25mL MTBE extrahiert und die vereinigte organische Phase mit 30mL Wasser und 30mL gesättigter NaCl-Lösung gewaschen.

Das Extrakt wird über Natriumsulfat getrocknet und das Lösemittel entfernt. Auswaage: 6,53g

1H-NMR: 7,2 ppm (dt,1 H, -CFH); 3,2-2,7 ppm (m, 4H, S-C$\mathbf{H}_2$-C$\mathbf{H}_2$-$SO_3$Na); Tabelle 1 zeigt die statische Oberflächenspannung und die CMC (critical micelle concentration) von erfindungsgemäßen Verbindungen.

**Tabelle 1:** statische Oberflächenspannungsmessung der oben beschriebenen Tenside als 0,1% wässrige Lösung in Dyn (mN/m)

| **Bsp** | 1c | 1d | 1e | 1f | 1g | 2b | 2d |
|---|---|---|---|---|---|---|---|
| **Dyn** | 20,4 | 20,5 | 27,0 | 20,0 | 17,0 | 16,0 | 20,2 |
| **Bsp** | 3b | 3c | 3d | 3e | 3f | 4 | |
| **Dyn** | 18,2 | 18,9 | 20,3 | 21,1 | 18,5 | 19,3(1%) | |

## Patentansprüche

1. Verbindungen der Formeln (I), (IIa), (V), (VI), (VII), (XIII), (XIII') oder (XV)

$$(R^1\text{-CHF-CF}_2\text{-Y-})_m\text{Spacer}(X)_n \qquad (I)$$

wobei in Formel (I)

$R^1$ = eine perfluorierte, ggf. Heteroatome enthaltende, lineare oder verzweigte, Alkylgruppe ist,
Spacer = eine gesättigte oder ungesättigte, verzweigte oder unverzweigte, ggf. Heteroatome enthaltende, Kohlenwasserstoffeinheit ist, wobei keine -O-O-Bindungen vorliegen, ist,
X = eine anionische, kationische, nicht-ionische oder amphotere hydrophile Gruppe,
Y = SO oder $SO_2$,
m = 1, 2, 3,4, 5 oder 6 und
n = 1, 2, 3 oder 4,

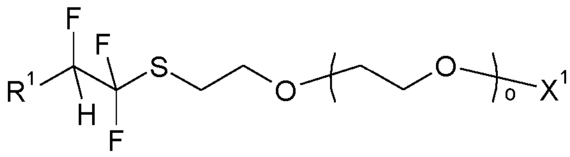

(IIa)

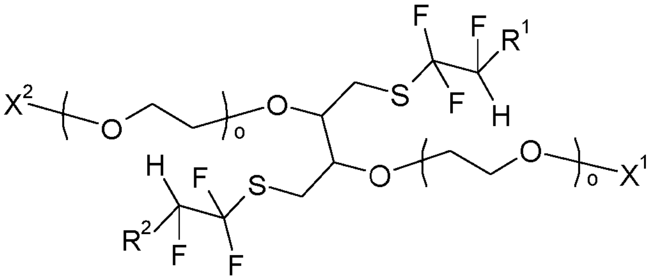

(V)

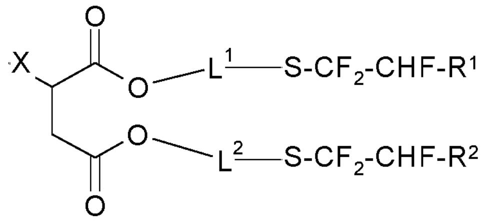

(VI)

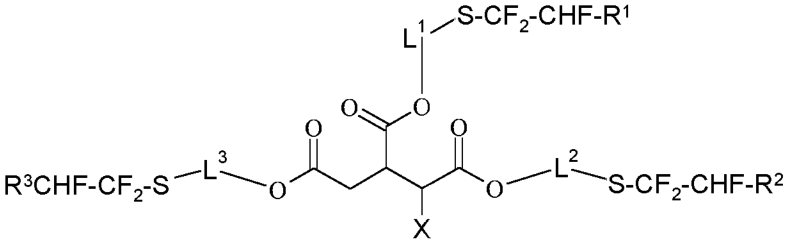

(VII)

wobei in den Formeln (IIa), (V), (VI) und VII)

$R^1$, $R^2$ und $R^3$ unabhängig voneinander eine fluorierte, ggf. Heteroatome enthaltende, lineare oder verzweigte, Alkylgruppe sind, o = 0-100, bevorzugt gleich 1-30 und 5-30, insbesondere 3, 5, 6, 10, 12, 15, 18, 20 oder 24, ist, $X^1$ und $X^2$ unabhängig voneinander eine anionische, kationische, nicht-ionische oder amphotere hydrophile Gruppe sind bzw. in den Formeln (IIa) und (V) auch gleich H sind, $L^1$, $L^2$ und $L^3$ unabhängig voneinander eine gesättigte oder ungesättigte, verzweigte oder unverzweigte, ggf. Heteroatome enthaltende, Kohlenwasserstoffeinheit sind, wobei keine -O-O-Bindungen vorliegen, insbesondere eine linear oder verzweigt C1-C6-Alkyl-gruppe,

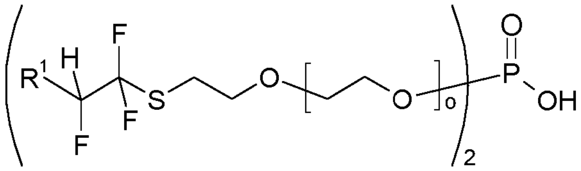

(XIII)

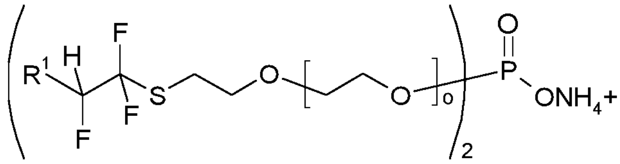

(XIII')

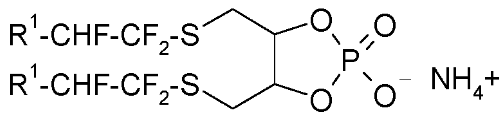

(XV)

wobei in den Formeln (XIII), (XIII') und (XV)
$R^1$ aus den Gruppen $CF_3-(CF_2)_{0-3}-$, $CF_3-(CF_2)_{0-3}-O-$, $CF_3-(CF_2)_{0-3}-O-(CF_2)_{1-3}-$, $CF_3-(CF_2)_{0-3}-O-(CF_2)_{1-3}-O-$, $CF_3-(CF_2)_{0-3}-O-(CF_2)_{1-3}-O-CF_2-$, $CF_3-(CF_2)_{0-3}-O-(CF_2-O)_{1-8}-$ und $CF_3-(CF_2)_{0-3}-O-(CF_2-O)_{1-8}-CF_2-$ausgewählt ist, und o gleich 1-30, insbesondere 3, 5, 6, 10, 12, 15, 18, 20 oder 24 ist,,
wobei die anionischen Gruppen X, $X^1$ und $X^2$ ausgewählt sind aus $-COO^-$, $-SO_3^-$, $-OSO_3^-$ , $-PO_3^{2-}$, $-OPO_3^{2-}$, $-OP(O)(O^-)O-$, $-(OCH_2CH_2)_s-O-(CH_2)_t-COO^-$, $-(OCH_2CH_2)_s-O-(CH_2)t\ -SO_3^-$, - $(OCH_2CH_2)_s-O-(CH_2)_t-OSO_3^-$, $-(OCH_2CH_2)_s-O-(CH_2)_t-PO_3^{2-}$, $-(OCH_2CH_2)_s-O-(CH_2)_t-OPO_3^{2-}$ oder aus den Formeln A bis C,

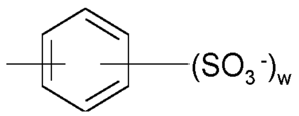

A

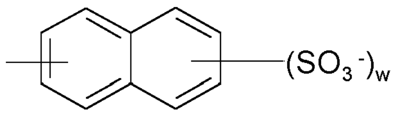

B

oder

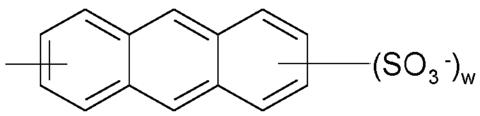

C

wobei s für eine ganze Zahl aus dem Bereich von 1 bis 1000 steht, t für eine ganze Zahl ausgewählt aus 1, 2, 3 oder 4 steht, und w für eine ganze Zahl ausgewählt aus 1, 2 oder 3 steht,
wobei das Gegenion ein einwertiges Kation, insbesondere $H^+$, ein Alkalimetall-Kation oder $NR_4^+$ ist, wobei R = H oder C1 - C6-Alkyl ist und alle R gleich oder verschieden sein können,
und die kationischen Gruppen X, $X^1$ und $X^2$ ausgewählt sind aus $-NR^1R^2R^{3+}Z^-$, $-PR^1R^2R^{3+}Z^-$,

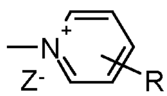

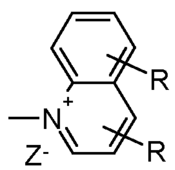

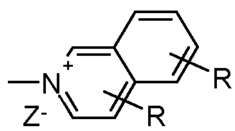

wobei R für H oder $C_{1-4}$-Alkyl in beliebiger Position steht,
$Z^-$ für $Cl^-$, $Br^-$, $I^-$, $CH_3SO_3^-$, $CF_3SO_3^-$, $CH_3PhSO_3^-$, $PhSO_3^-$ steht,
$R^1$, $R^2$ und $R^3$ jeweils unabhängig voneinander für H, $C_{1-30}$-Alkyl, Ar oder $-CH_2Ar$ stehen, und
Ar für einen unsubstituierten oder ein- oder mehrfach substituierten aromatischen Ring oder kondensierte Ringsysteme mit 6 bis 18 C-Atomen steht, worin auch ein oder zwei CH-Gruppen durch N ersetzt sein können, und die nicht-ionischen Gruppen X, $X^1$ und $X^2$ ausgewählt sind aus linearem oder verzweigtem Alkyl, wobei ein oder mehrere nicht benachbarte C-Atome durch O, S, und/oder N ersetzt sind, -OH, -SH, $-O-(Glycosid)_{o'}$,

-S-(Glycosid)$_{o'}$, -$OCH_2$-CHOH-$CH_2$-OH , -$OCH_2$Ar(-NCO)$_{p'}$, -OAr(-NCO)$_{p'}$, Aminoxid,

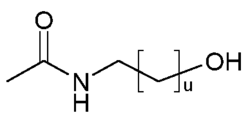
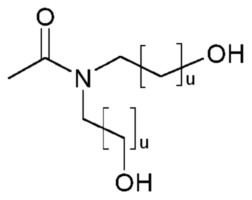
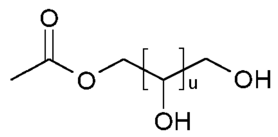

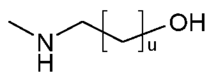
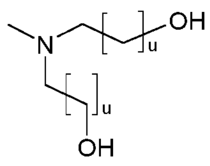
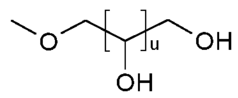

worin u für eine ganze Zahl aus dem Bereich von 1 bis 6, bevorzugt 1 bis 4 steht,
o'für eine ganze Zahl aus dem Bereich von 1 bis 10 steht,
p'für 1 oder 2 steht,
Ar für einen unsubstituierten, ein- oder mehrfach substituierten aromatischen Ring oder kondensierte Ringsysteme mit 6 bis 18 C-Atomen steht, worin auch ein oder zwei CH-Gruppen durch C=O ersetzt sein können und,
Glycosid für ein verethertes Kohlenhydrat, vorzugsweise für ein monodi-, tri- oder oligo-Glucosid steht,
und die amphoteren Gruppen X, $X^1$ und $X^2$ ausgewählt sind aus den funktionellen Gruppen der Acetyldiamine, der N-Alkylaminosäuren, der N-Alkylaminosulfonsäuren, der Betaine, der Sulfobetaine, bzw. entsprechender Derivate, insbesondere ausgewählt aus, wobei M für H oder ein Alkalimetall-Ion, vorzugsweise $Li^+$, $Na^+$ oder $K^+$ steht:

| |
|---|
| 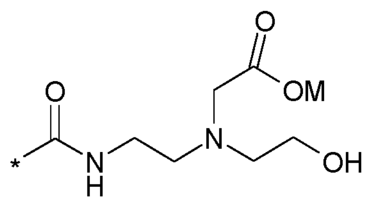 |
| 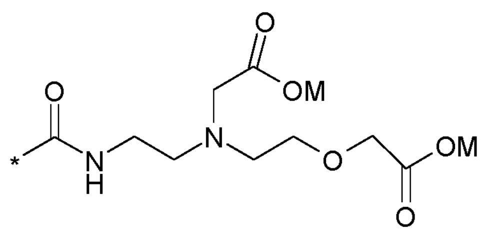 |
| 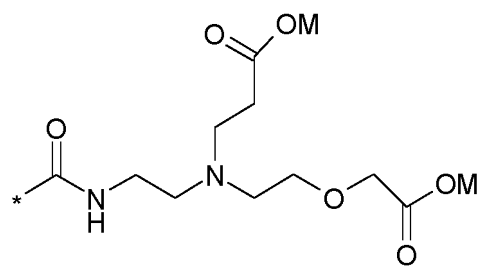 |
| -NH-$CH_2$-COOM; -NH-$CH_2$-$CH_2$-COOM |

(fortgesetzt)

| |
|---|
| 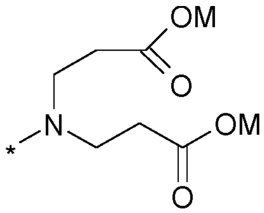 |
| -[(C(=O)-NH-$(CH_2)_{(1-8)}]_{(0\ oder\ 1)}$-$N^+R^1R^2$-$CH_2$-$COO^-$, wobei $R^1$ und $R^2$ jeweils unabhängig voneinander für einen C1-8-Alkylrest, vorzugsweise Methyl oder Ethyl, stehen |
| -C(=O)-NH-$(CH_2)_{1-3}$-$N^+R^1R^2$-$CH_2$-CH(OH)-$CH_2$-$(O)_{(0\ oder\ 1)}$-(S oder P)$O_3^-$, wobei $R^1$ und $R^2$ jeweils unabhängig voneinander für einen C1-8-Alkylrest, vorzugsweise Methyl oder Ethyl, stehen. |

**2.** Verbindungen gemäß Anspruch 1, **dadurch gekennzeichnet, dass**

m = 2-4, insbesondere 2-3, und
n = 1 oder 2, ist.

**3.** Verbindungen gemäß einem oder mehreren der Ansprüche 1 bis 2, **dadurch gekennzeichnet, dass** die fluorierte Gruppe $R^1$ in Formel (I) aus den Gruppen $CF_3$-$(CF_2)_{0-3}$-, $CF_3$-$(CF_2)_{0-3}$-O-, $CF_3$-$(CF_2)_{0-3}$-O-$(CF_2)_{1-3}$-, $CF_3$-$(CF_2)_{0-3}$-O-$(CF_2)_{1-3}$-O-, $CF_3$-$(CF_2)_{0-3}$-O-$(CF_2)_{1-3}$-O-$CF_2$-, $CF_3$-$(CF_2)_{0-3}$-O-$(CF_2$-$O)_{1-8}$- und $CF_3$-$(CF_2)_{0-3}$-O-(C-$F_2$-$O)_{1-8}$-$CF_2$-ausgewählt ist.

**4.** Verbindungen gemäß einem oder mehreren der Ansprüche 1 bis 3, **dadurch gekennzeichnet, dass** sie einer der Formeln (IIa-c) oder (V) bis (VII) entsprechen:

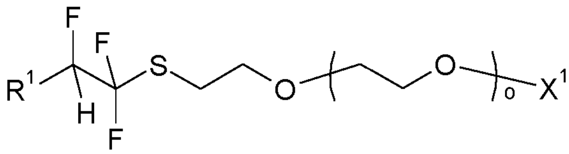

(IIa)

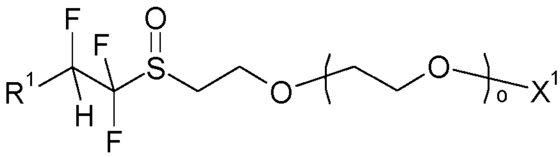

(IIb)

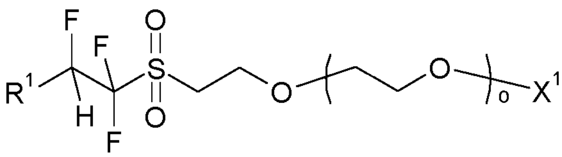

(IIc)

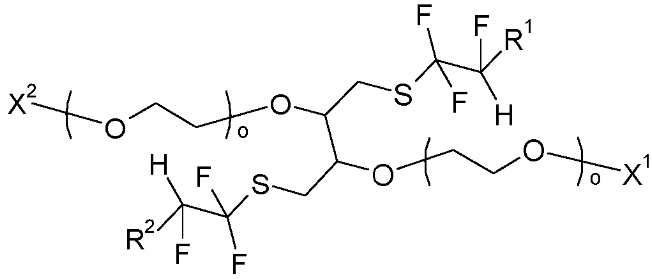

(V)

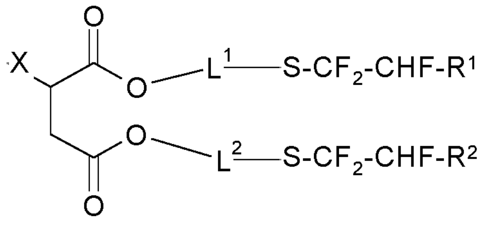

(VI)

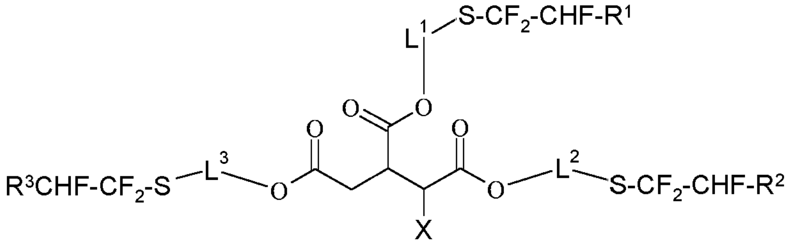

(VII)

worin $R^1$, $R^2$ und $R^3$ die in Anspruch 1 oder 3 angegebene Bedeutung haben, o = 1-30 und 5-30, insbesondere 3, 5, 6, 10, 12, 15, 18, 20 oder 24, ist, $X^1$ und $X^2$ unabhängig voneinander eine anionische, kationische, nicht-ionische oder amphotere hydrophile Gruppe wie in Anspruch 1 definiert sind bzw. in den Formeln (IIa), (IIb), (IIc) und (V) auch gleich H sind, $L^1$, $L^2$ und $L^3$ unabhängig voneinander eine linear oder verzweigt C1-C6-Alkylgruppe.

5. Verbindungen gemäß einem oder mehreren der Ansprüche 1 bis 4, **dadurch gekennzeichnet, dass** sie einer der Formeln (XIII) bis (XVIII) entsprechen:

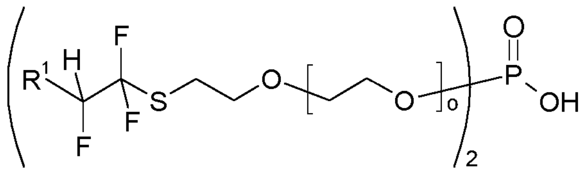

(XIII)

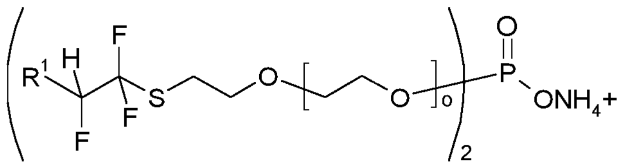

(XIII‘)

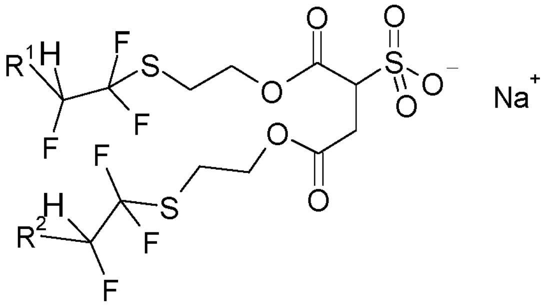

(XIV)

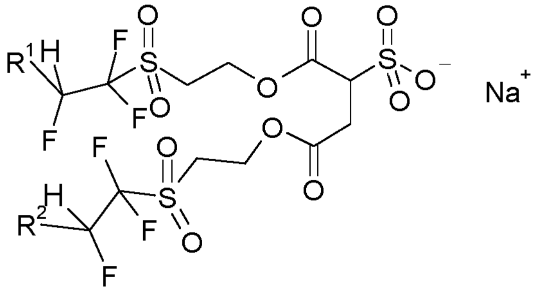

(XIVa)

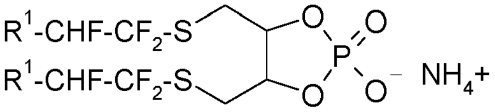

(XV)

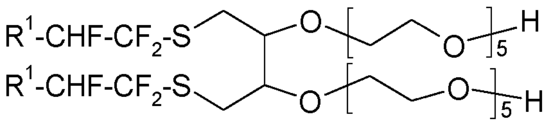

(XVI)

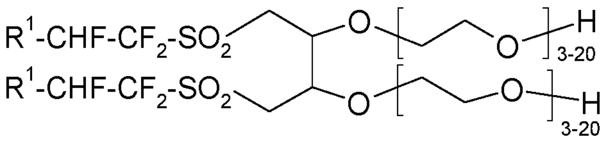

(XVIa)

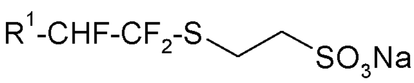

(XVII)

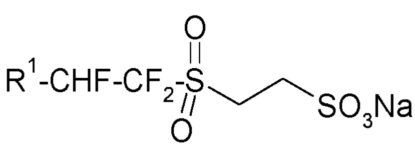

(XVIIa)

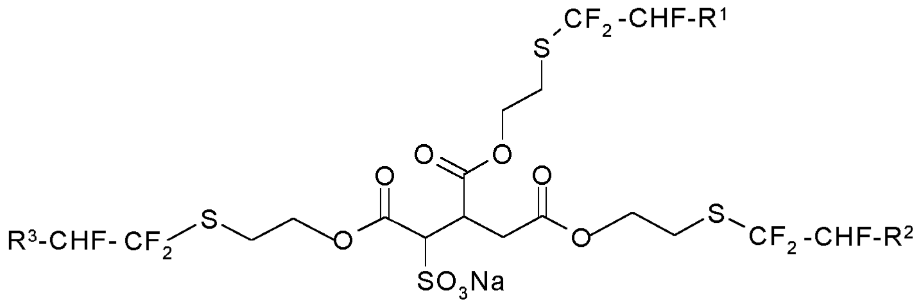

(XVIII)

wobei $R^1$, $R^2$ und $R^3$ unabhängig voneinander ausgewählt sind aus den Gruppen $CF_3$-$(CF_2)_{0-3}$-, $CF_3$-$(CF_2)_{0-3}$-O-, $CF_3$-$(CF_2)_{0-3}$-O-$(CF_2)_{1-3}$-, $CF_3$-$(CF_2)_{0-3}$-O-$(CF_2)_{1-3}$-O-, $CF_3$-$(CF_2)_{0-3}$-O-$(CF_2)_{1-3}$-O-$CF_2$-, $CF_3$-$(CF_2)_{0-3}$-O-(C-$F_2$-O$)_{1-8}$- und $CF_3$-$(CF_2)_{0-3}$-O-$(CF_2$-O$)_{1-8}$-$CF_2$-, o gleich 3, 5, 6, 10, 12, 15, 18, 20 oder 24 ist..

**6.** Verbindungen gemäß einem oder mehreren der Ansprüche 1 bis 5, **dadurch gekennzeichnet, dass** sie einer der Formeln (XIX) bis (XXIX) entsprechen:

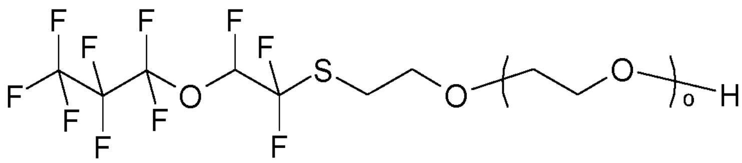

(XIXa)

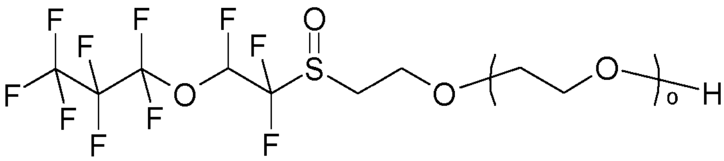

(XIXb)

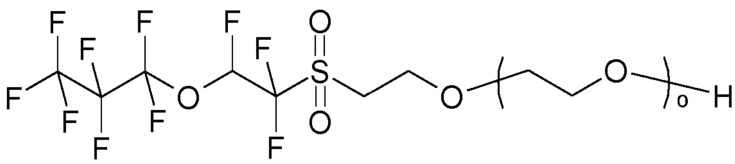

(XIXc)

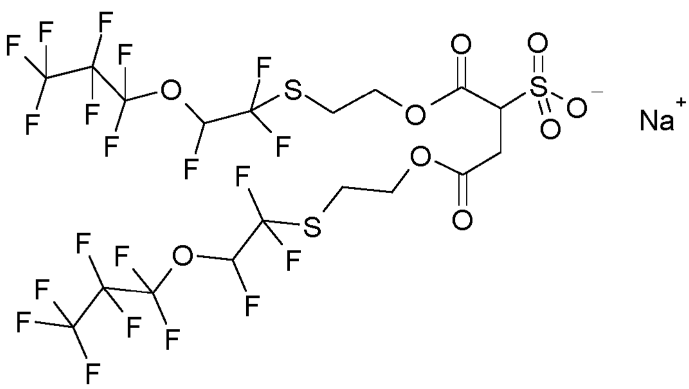

(XX)

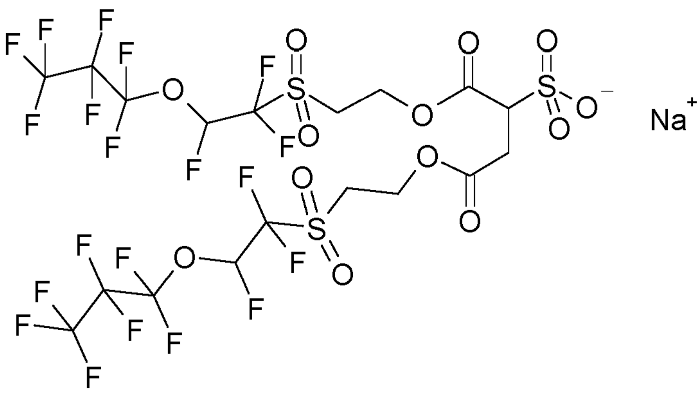

(XXa)

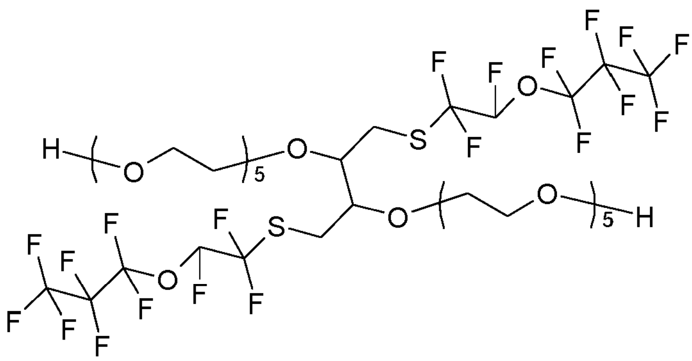

(XXI)

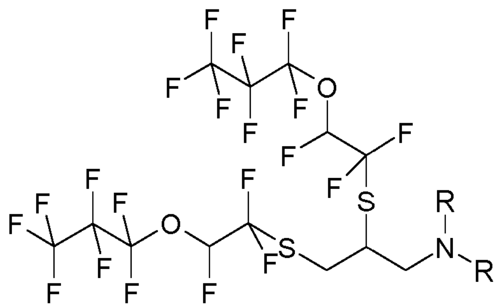

(XXII)

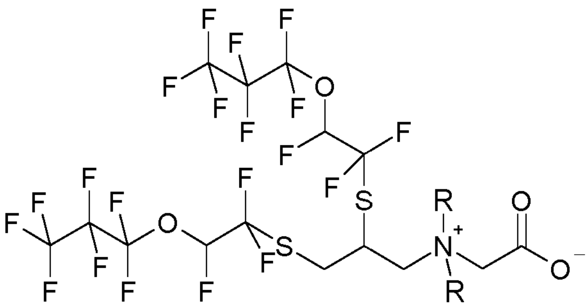

(XXIII)

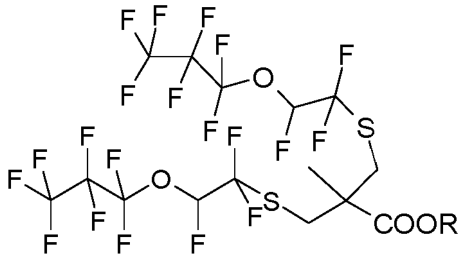

(XXIV)

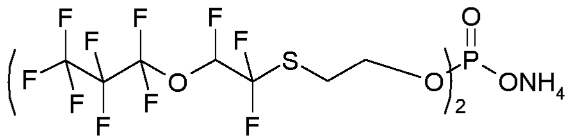

(XXV)

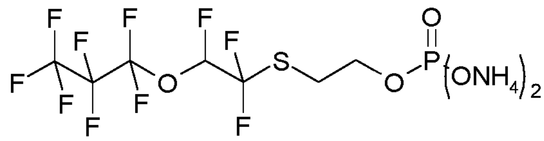

(XXVI)

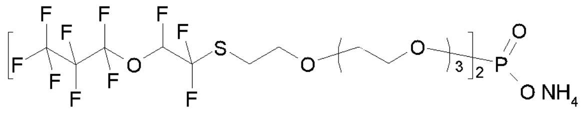

(XXVII)

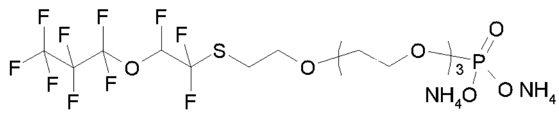

(XXVIII)

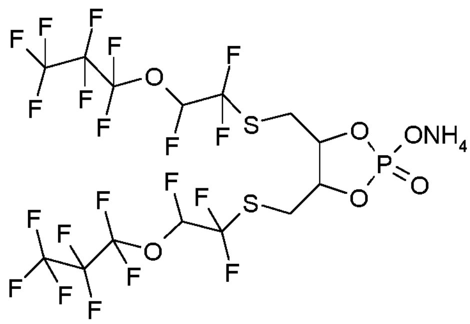

(XXIX)

wobei o = 0, 10 oder 18 und R = Methyl oder Ethyl.

**7.** Verwendung von Verbindungen gemäß einem oder mehreren der Ansprüche 1 bis 6 als Additive in Farben, Lacken, Druckfarben, Schutzanstrichen, Spezialcoatings in elektronischen oder optischen Anwendungen, Photolacken, Top Antireflective Coatings oder Bottom Antireflective Coatings, Entwicklerlösungen und Waschlösungen und Photoresists für photolithograqphische Verfahren, kosmetischen Produkten, Agrochemikalien, Bodenpolituren, photographischen Beschichtungen oder Beschichtungen optischer Elemente.

**8.** Mittel enthaltend eine Verbindungen gemäß einem oder mehreren der Ansprüche 1 bis 6 und einen für den jeweiligen Verwendungszweck geeigneten Träger sowie ggf. weitere spezifische Aktivstoffe.

**9.** Mittel nach Anspruch 8, **dadurch gekennzeichnet, dass** es sich bei dem Mittel um Farb- und Lackzubereitungen,

Feuerlöschmittel, Schmierstoffe, Wasch- und Reinigungsmittel, Enteiser, Entwicklerlösungen und Waschlösungen und Photoresists für photolithograqphische Verfahren, kosmetische Produkte, Agrochemikalien, Bodenpolituren oder Hydrophobiermittel zur Textilausrüstung oder Glasbehandlung handelt.

## Claims

**1.** Compounds of formulae (I), (IIa), (V), (VI), (VII), (XIII), (XIII') or (XV)

$$(R^1\text{-CHF-CF}_2\text{-Y-})_m\text{spacer}(X)_n \qquad (I)$$

wherein in formula (I)

$R^1$ = a perfluorinated, linear or branched, alkyl group optionally containing heteroatoms,
spacer = a saturated or unsaturated, branched or unbranched, hydrocarbon unit optionally containing heteroatoms, wherein no O-O bonds are present,
X = an anionic, cationic, nonionic or amphoteric hydrophilic group,
Y = SO or $SO_2$,
m = 1, 2, 3, 4, 5 or 6 and
n = 1, 2, 3 or 4,

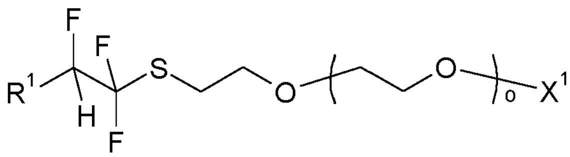

(IIa)

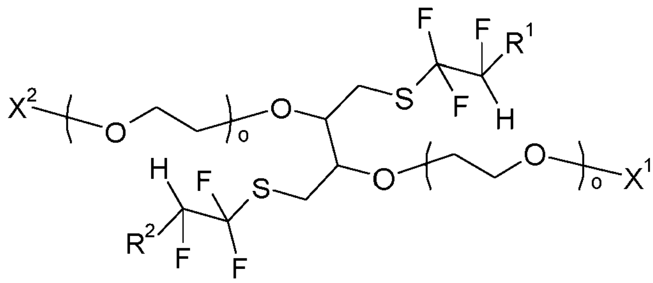

(V)

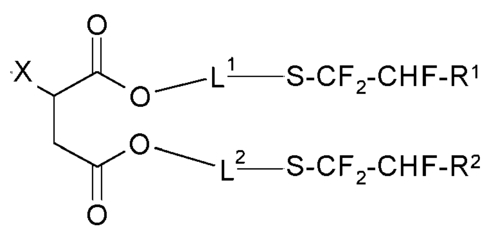

(VI)

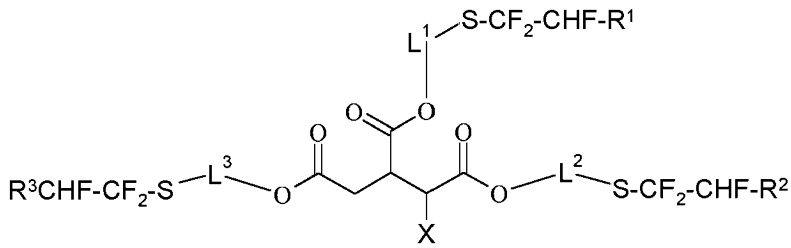

(VII)

wherein in formulae (IIa), (V), (VI) and (VII)

$R^1$, $R^2$ and $R^3$ are independently a fluorinated, linear or branched, alkyl group optionally containing heteroatoms, o = 0-100, preferably 1-30 and 5-30, especially 3, 5, 6, 10, 12, 15, 18, 20 or 24, $X^1$ and $X^2$ are independently an anionic, cationic, nonionic or amphoteric hydrophilic group or in formulae (IIa) and (V) are also H, $L^1$, $L^2$ and $L^3$ are independently a saturated or unsaturated, branched or unbranched, hydrocarbon unit optionally containing heteroatoms, wherein no -O-O bonds are present, especially a linear or branched C1-C6-alkyl group,

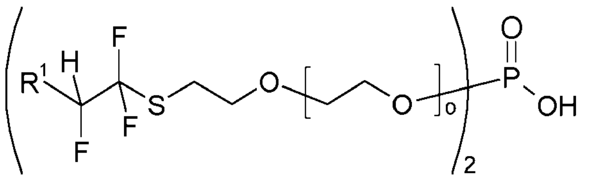

(XIII)

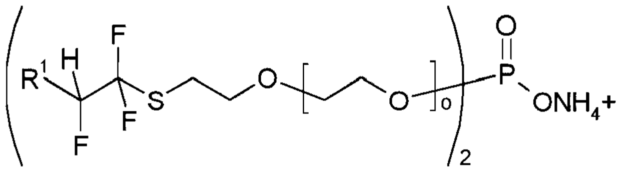

(XIII')

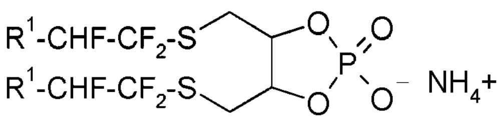

(XV)

wherein in formulae (XIII), (XIII') and (XV)
$R^1$ is selected from the groups $CF_3-(CF_2)_{0-3}-$, $CF_3-(CF_2)_{0-3}-O-$, $CF_3-(CF_2)_{0-3}-O-(CF_2)_{1-3}-$, $CF_3-(CF_2)_{0-3}-O-(CF_2)_{1-3}-O-$, $CF_3-(CF_2)_{0-3}-O-(CF_2)_{1-3}-O-CF_2-$, $CF_3-(CF_2)_{0-3}-O-(CF_2-O)_{1-8}-$ and $CF_3-(CF_2)_{0-3}-O-(CF_2-O)_{1-8}-CF_2-$ and o is 1 30, especially 3, 5, 6, 10, 12, 15, 18, 20 or 24,
wherein the anionic groups X, $X^1$ and $X^2$ are selected from $-COO^-$, $-SO_3^-$, $-OSO_3$ $-PO_3^{2-}$, $-OPO_3^{2-}$, $-OP(O)(O^-)O-$, $-(OCH_2CH_2)_s-O-(CH_2)_t-COO^-$, - $(OCH_2CH_2)_s-O-(CH_2)_t$ $-SO_3^-$, $-(OCH_2CH_2)_s-O-(CH_2)_t-OSO_3^-$, $-(OCH_2CH_2)_s-O-(CH_3)-PO_3^{2-}$, $-(OCH_2CH_2)_s-O-(CH_2)_t-OPO_3^{2-}$ or from the formulae A to C,

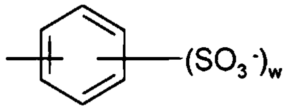

A

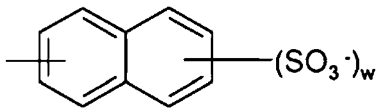

B

or

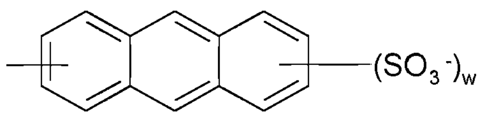

C

wherein s is an integer from the range from 1 to 1000, t is an integer selected from 1, 2, 3 or 4 and w is an integer selected from 1, 2 or 3, wherein the counterion is a monovalent cation, especially $H^+$, an alkali metal cation or $NR_4^+$, wherein R = H or C1-C6-alkyl and all R may be identical or different,
and the cationic groups X, $X^1$ and $X^2$ are selected from $-NR^1R^2R^{3+}Z^-$, $-PR^1R^2R^{3+}Z^-$,

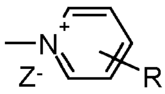 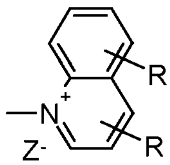 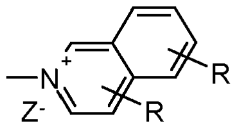

wherein R represents H or $C_{1-4}$-alkyl in any position,

$Z^-$ represents $Cl^-$, $Br^-$, $I^-$, $CH_3SO_3^-$, $CF_3SO_3^-$, $CH_3PhSO_3^-$, $PhSO_3^-$, $R^1$, $R^2$ and $R^3$ each independently represent H, $C_{1-30}$-alkyl, Ar or $-CH_2Ar$ and

Ar represents an unsubstituted or mono- or polysubstituted aromatic ring or condensed ring systems having 6 to 18 C atoms in which one or two CH groups may also be replaced by N,

and the nonionic groups X, $X^1$ and $X^2$ are selected from linear or branched alkyl, wherein one or more non-adjacent C atoms are replaced by O, S, and/or N, -OH, -SH, -O-(glycoside)$_{o'}$, -S-(glycoside)$_{o'}$, $-OCH_2-CHOH-CH_2-OH$, $-OCH_2Ar(-NCO)_{p'}$, $-OAr(-NCO)_{p'}$, amine oxide,

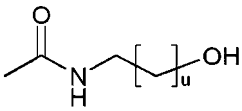 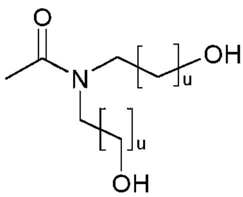 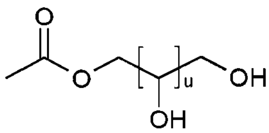

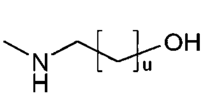 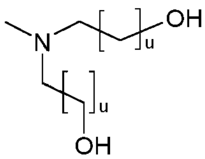 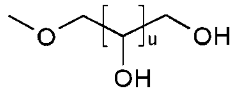

wherein u represents an integer from the range from 1 to 6, preferably 1 to 4,

o' represents an integer from the range from 1 to 10,

p' represents 1 or 2,

Ar represents an unsubstituted, mono- or polysubstituted aromatic ring or condensed ring systems having 6 to 18 C atoms in which one or two CH groups may also be replaced by C=O,

glycoside represents an etherified carbohydrate, preferably a mono- di-, tri- or oligo-glucoside,

and the amphoteric groups X, $X^1$ and $X^2$ are selected from the functional groups of acetyldiamines, N-alkylaminoacids, N-alkylaminosulfonic acids, betaines, sulfobetaines and corresponding derivatives, especially selected from, wherein M represents H or an alkali metal ion, preferably $Li^+$, $Na^+$ or $K^+$:

| |
|---|
| 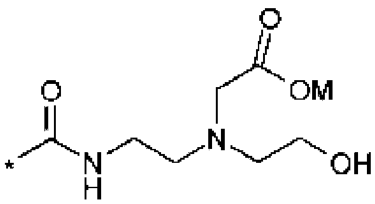 |
| 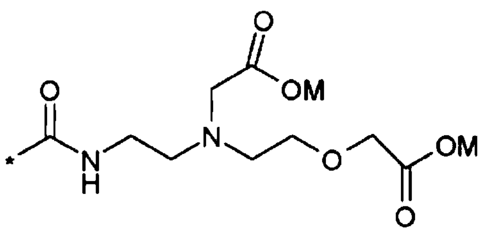 |

(fortgesetzt)

| |
|---|
| 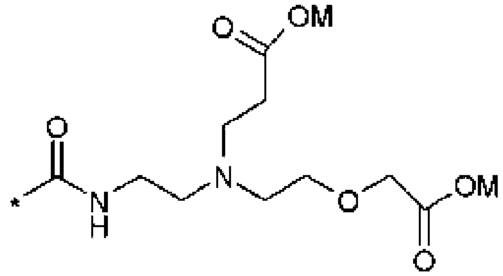 |
| $-NH-CH_2-COOM$; $-NH-CH_2-CH_2-COOM$ |
| 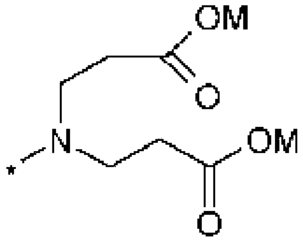 |
| $-[(C(=O)-NH-(CH_2)_{(1-8)}]_{(0\ or\ 1)}-N^+R^1R^2-CH_2-COO^-$, wherein $R^1$ and $R^2$ each independently represent a C1-8-alkyl radical, preferably methyl or ethyl, |
| $-C(=O)-NH-(CH_2)_{1-3}-N^+R^1R^2-CH_2-CH(OH)-CH_2-(O)_{(0\ or\ 1)}-(S\ or\ P)O_3^-$, wherein $R^1$ and $R^2$ each independently represent a C1-8-alkyl radical, preferably methyl or ethyl. |

**2.** Compounds according to Claim 1, **characterized in that**

m = 2-4, especially 2-3, and
n = 1 or 2.

**3.** Compounds according to one or more of Claims 1 to 2, **characterized in that** the fluorinated group $R^1$ in formula (I) is selected from the groups $CF_3-(CF_2)_{0-3}-$, $CF_3-(CF_2)_{0-3}-O-$, $CF_3-(CF_2)_{0-3}-O-(CF_2)_{1-3}-$, $CF_3-(CF_2)_{0-3}-O-(CF_2)_{1-3}-O-$, $CF_3-(CF_2)_{0-3}-O-(CF_2)_{1-3}-O-CF_2-$, $CF_3-(CF_2)_{0-3}-O-(CF_2-O)_{1-8}-$ and $CF_3-(CF_2)_{0-3}-O-(CF_2-O)_{1-8}-CF_2-$.

**4.** Compounds according to one or more of Claims 1 to 3, **characterized in that** they conform to one of formulae (IIa-c) or (V) to (VII):

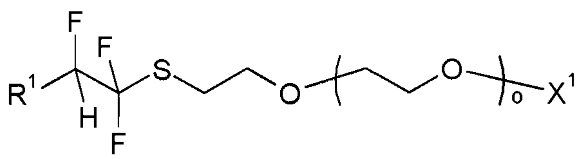

(IIa)

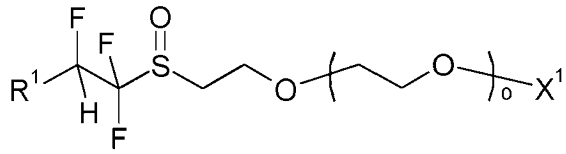

(IIb)

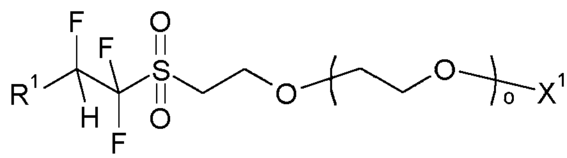

(IIc)

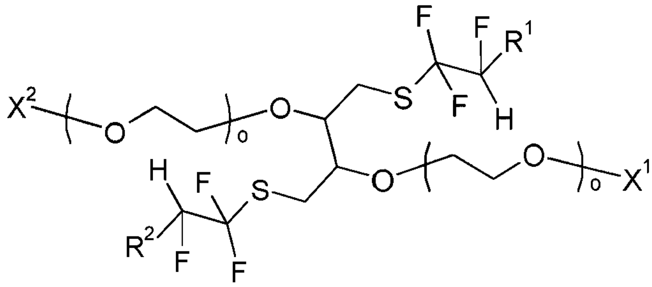

(V)

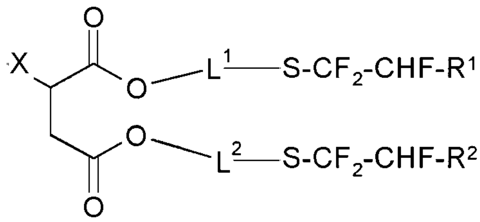

(VI)

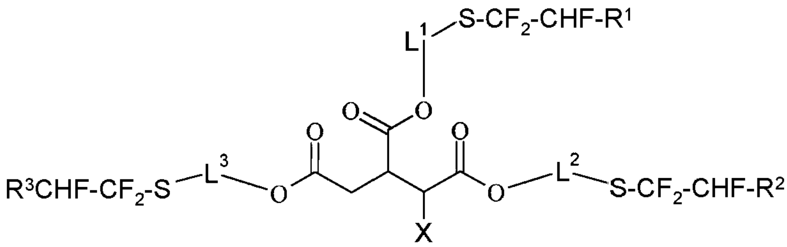

(VII)

wherein $R^1$, $R^2$ and $R^3$ are as defined in claims 1 or 3 o = 1-30 and 5-30, especially 3, 5, 6, 10, 12, 15, 18, 20 or 24, $X^1$ and $X^2$ are independently an anionic, cationic, nonionic or amphoteric hydrophilic group as defined in Claim 1 or in formulae (IIa), (IIb), (IIc) and (V) are also H, $L^1$, $L^2$ and $L^3$ are independently a linear or branched C1-C6 alkyl group.

**5.** Compounds according to one or more of Claims 1 to 4, **characterized in that** they conform to one of formulae (XIII) to (XVIII):

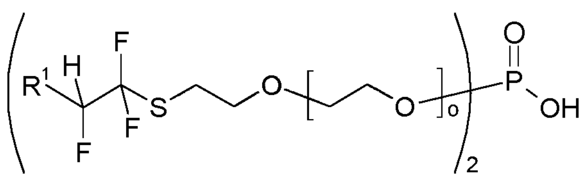

(XIII)

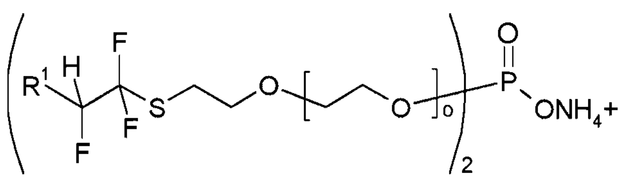

(XIII')

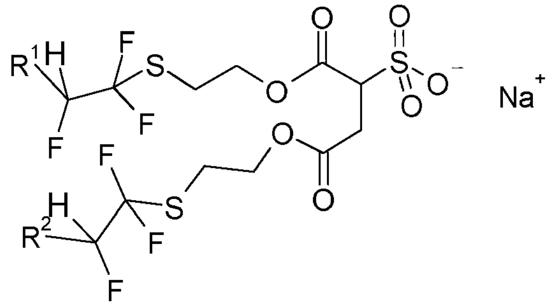

(XIV)

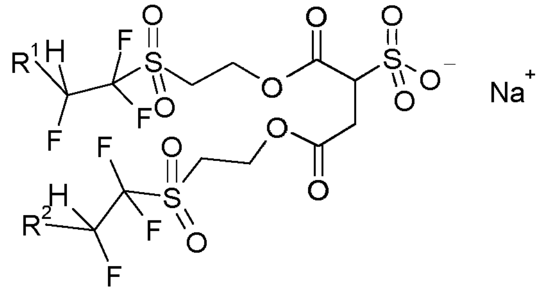

(XIVa)

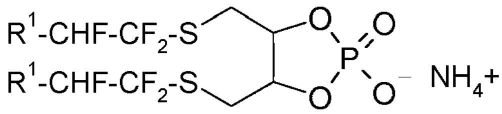

(XV)

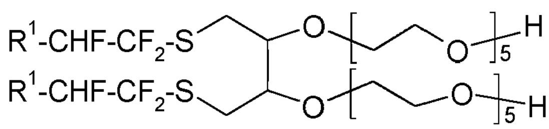

(XVI)

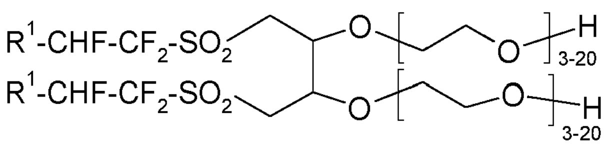

(XVIa)

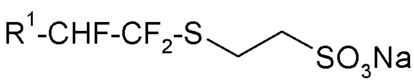

(XVII)

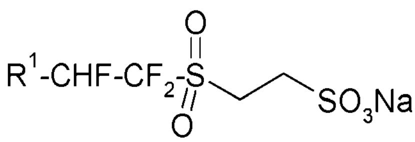

(XVIIa)

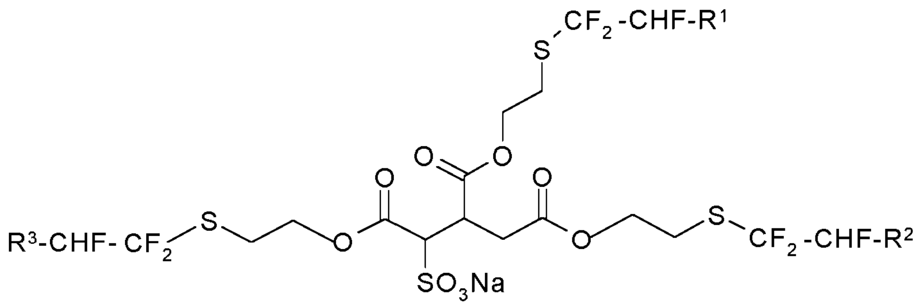

(XVIII)

wherein $R^1$, $R^2$ and $R^3$ are independently selected from the groups $CF^3$-$(CF_2)_{0-3}$-, $CF_3$-$(CF_2)_{0-3}$-O-, $CF_3$-$(CF_2)_{0-3}$-O-$(CF_2)_{1-3}$-, $CF_3$-$(CF_2)_{0-3}$-O-$(CF_2)_{1-3}$-O-, $CF_3$-$(CF_2)_{0-3}$-O-$(CF_2)_{1-3}$-O-$CF_2$-, $CF_3$-$(CF_2)_{0-3}$-O-$(CF_2$-$O)_{1-8}$- and $CF_3$-$(CF_2)_{0-3}$-O-$(CF_2$-$O)_{1-8}$-$CF_2$- and o is 3, 5, 6, 10, 12, 15, 18, 20 or 24.

**6.** Compounds according to one or more of Claims 1 to 5, **characterized in that** they conform to one of formulae (XIX) to (XXIX):

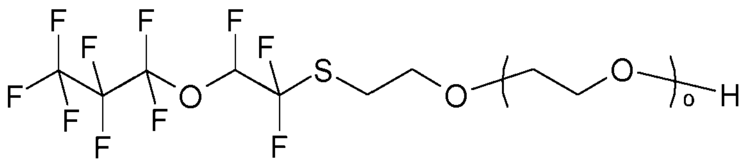

(XIXa)

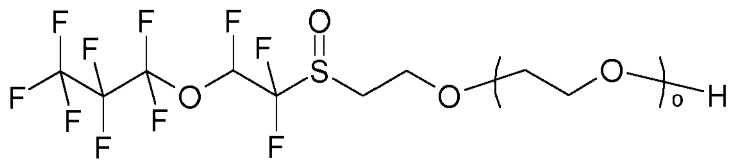

(XIXb)

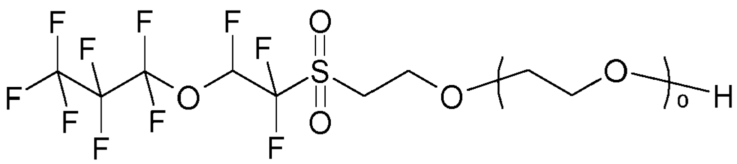

(XIXc)

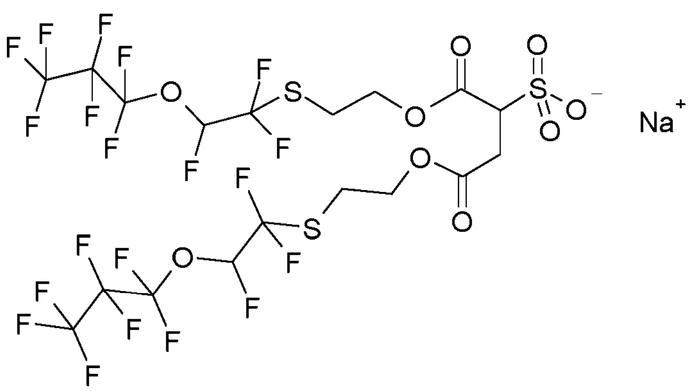

(XX)

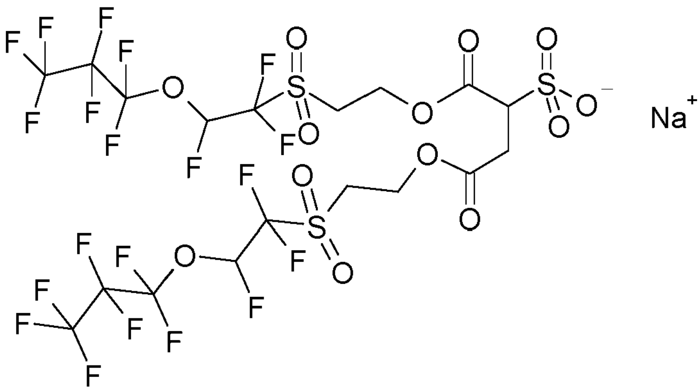

(XXa)

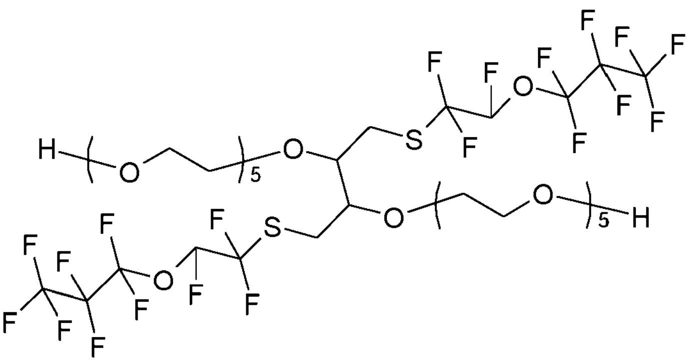

(XXI)

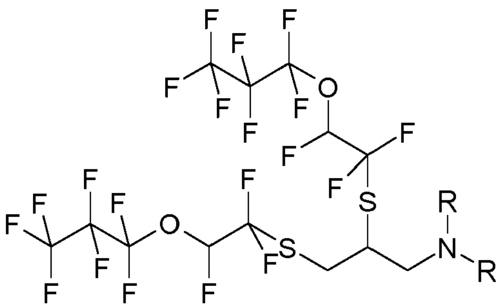

(XXII)

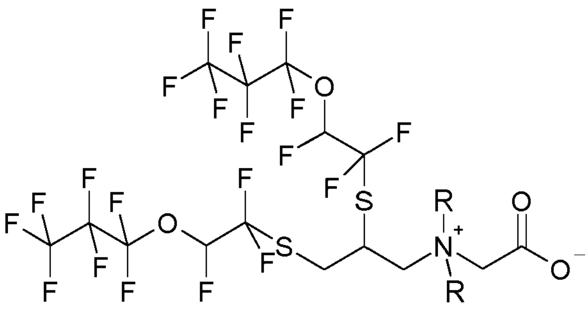

(XXIII)

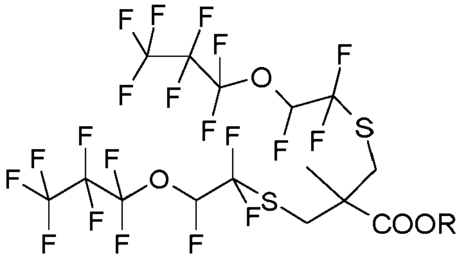
(XXIV)

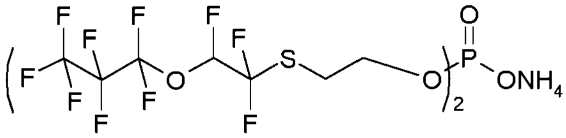
(XXV)

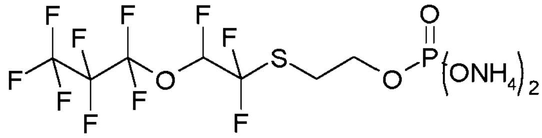
(XXVI)

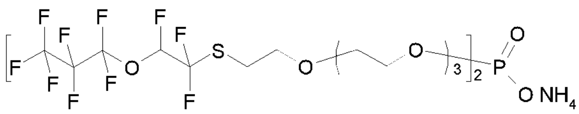
(XXVII)

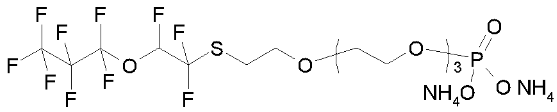
(XXVIII)

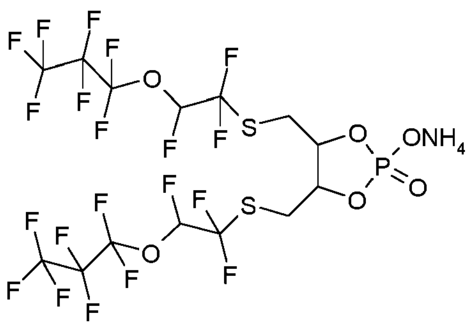
(XXIX)

wherein o = 0, 10 or 18 and R = methyl or ethyl.

**7.** Use of compounds according to one or more of Claims 1 to 6 as additives in paints, lacquers, printing inks, protective coatings, special coatings in electronic or optical applications, photoresists, top antireflective coatings or bottom antireflective coatings, developer solutions and washing solutions and photoresists for photolithographic processes, cosmetic products, agrochemicals, floor polishes, photographic coatings or coatings of optical elements.

**8.** Composition containing a compound according to one or more of Claims 1 to 6 and a carrier suitable for the respective use and optionally further specific active substances.

**9.** Composition according to Claim 8, **characterized in that** the composition comprises paint and lacquer preparations, fire extinguishants, lubricants, washing and cleaning compositions, de-icers, developer solutions and washing

solutions and photoresists for photolithographic processes, cosmetic products, agrochemicals, floor polishes or hydrophobic agents for textile finishing or glass treatment.

## Revendications

**1.** Composés des formules (I), (IIa), (V), (VI), (VII), (XIII), (XIII') ou (XV)

$$(R^1\text{-CHF-CF}_2\text{-Y-})_m\text{écarteur}(X)_n \qquad (I)$$

où, dans la formule (I)

$R^1$ = un groupe alkyle linéaire ou ramifié, perfluoré, contenant le cas échéant des hétéroatomes,
écarteur = un motif hydrocarboné saturé ou insaturé, ramifié ou non ramifié, contenant le cas échéant des hétéroatomes, ne présentant pas de liaisons -O-O-,
X = un groupe anionique, cationique, non ionique ou amphotère hydrophile,
Y = SO ou $SO_2$,
m = 1, 2, 3, 4, 5 ou 6 et
n = 1, 2, 3 ou 4,

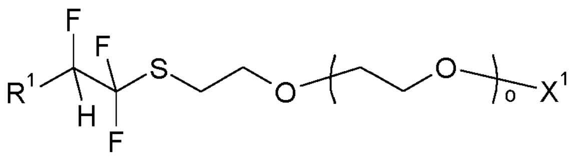

(IIa)

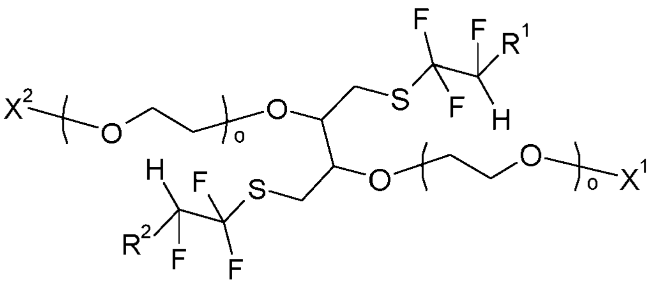

(V)

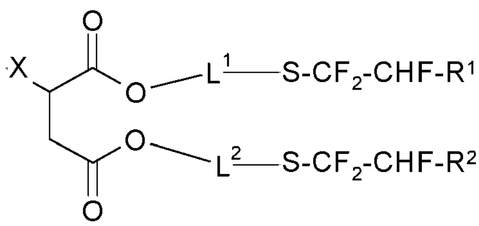

(VI)

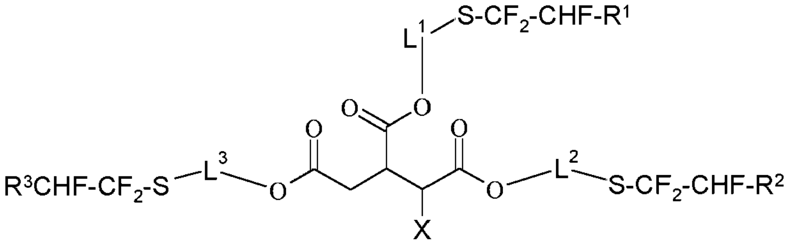

(VII)

où, dans les formules (IIa), (V), (VI) et (VII)
$R^1$, $R^2$ et $R^3$ représentent, indépendamment les uns des autres, un groupe alkyle linéaire ou ramifié, fluoré,

contenant le cas échéant des hétéroatomes, o = 0-100, de préférence 1-30 et 5-30, en particulier 3, 5, 6, 10, 12, 15, 18, 20 ou 24 ; $X^1$ et $X^2$ représentent, indépendamment l'un de l'autre, un groupe anionique, cationique, non ionique ou amphotère hydrophile ou, selon le cas, dans les formules (IIa) et (V) représentent également H ; $L^1$, $L^2$ et $L^3$ représentent, indépendamment les uns des autres, un motif hydrocarbone saturé ou insaturé, ramifié ou non ramifié, contenant le cas échéant des hétéroatomes, ne présentant pas de liaisons -O-O-, en particulier un groupe C1-C6-alkyle linéaire ou ramifié,

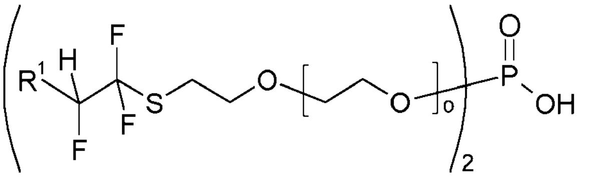

(XIII)

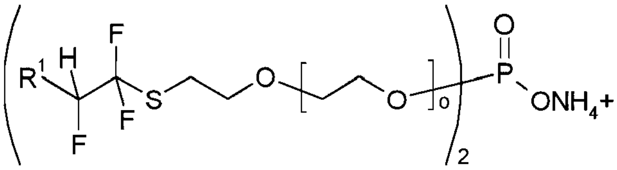

(XIII')

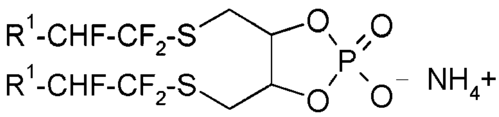

(XV)

où, dans les formules (XIII), (XIII') et (XV)
$R^1$ est choisi parmi les groupes $CF_3$-$(CF_2)_{0-3}$-, $CF_3$-$(CF_2)_{0-3}$-O-, $CF_3$-$(CF_2)_{0-3}$-O-$(CF_2)_{1-3}$-, $CF_3$-$(CF_2)_{0-3}$-O-$(CF_2)_{1-3}$-O-, $CF_3$-$(CF_2)_{0-3}$-O-$(CF_2)_{1-3}$-O-$CF_2$-, $CF_3$-$(CF_2)_{0-3}$-O-$(CF_2$-O$)_{1-8}$- et $CF_3$-$(CF_2)_{0-3}$-O-$(CF_2$-O$)_{1-8}$-$CF_2$- et o vaut 1-30, en particulier 3, 5, 6, 10, 12, 15, 18, 20 ou 24,
où les groupes anioniques X, $X^1$ et $X^2$ sont choisis parmi -$COO^-$, -$SO_3^-$, - $OSO_3^-$, -$PO_3^{2-}$, -$OPO_3^{2-}$, -OP(O)($O^-$)O-, -$(OCH_2CH_2)_s$-O-$(CH_2)_t$-$COO^-$, - $(OCH_2CH_2)_s$-O-$(CH_2)_t$-$SO_3^-$, -$(OCH_2CH_2)_s$-O-$(CH_2)_t$-$OSO_3^-$, -$(OCH_2CH_2)_s$-O-$(CH_2)_t$-$PO_3^{2-}$, -$(OCH_2CH_2)_s$-O-$(CH_2)_t$-$OPO_3^{2-}$ ou parmi les formules A à C,

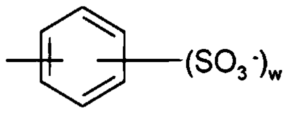

A

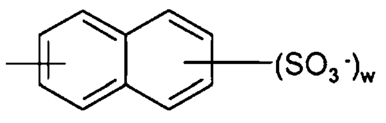

B

ou

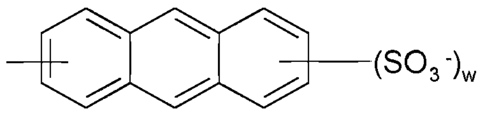

C

où s représente un nombre entier de la plage de 1 à 1000, t représente un nombre entier choisi parmi 1, 2, 3 ou 4 et w représente un nombre entier choisi parmi 1, 2 ou 3,
où le contre-ion est un cation monovalent, en particulier $H^+$, un cation de métal alcalin ou $NR_4^+$, où R = H ou C1-C6-alkyle et tous les R peuvent être identiques ou différents,
et les groupes cationiques X, $X^1$ et $X^2$ sont choisis parmi -$NR^1R^2R^{3+}Z^-$, - $PR^1R^2R^{3+}Z^-$,

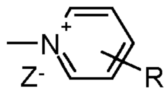 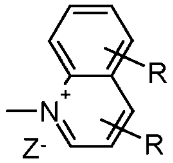 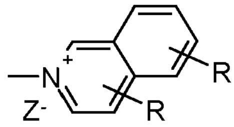

où R représente H ou $C_{1-4}$-alkyle dans une position quelconque,
$Z^-$ représente $Cl^-$, $Br^-$, $I^-$, $CH_3SO_3^-$, $CF_3SO_3^-$, $CH_3PhSO_3^-$, $PhSO_3^-$, $R^1$, $R^2$ et $R^3$ représentent, à chaque fois indépendamment les uns des autres, H, $C_{1-30}$-alkyle, Ar ou $-CH_2Ar$ et
Ar représente un cycle aromatique non substitué ou monosubstitué ou polysubstitué ou un système cyclique condensé comprenant 6 à 18 atomes de carbone, dans lequel un ou deux groupes CH peuvent également être remplacés par N,
et les groupes non ioniques X, $X^1$ et $X^2$ sont choisis parmi alkyle linéaire ou ramifié, un ou plusieurs atomes de C non adjacents étant remplacés par O, S et/ou N, -OH, -SH, -O-(glycoside)$_{o'}$, -S-(glycoside)$_{o'}$, $-OCH_2-CHOH-CH_2-OH$, $-OCH_2Ar(-NCO)_{p'}$, $-OAr(-NCO)_{p'}$, oxyde d'amine,

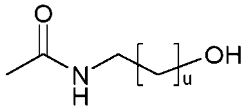 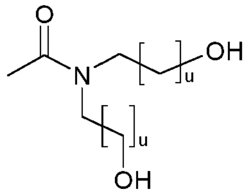 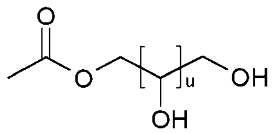

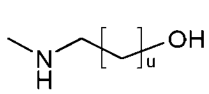 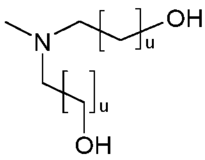 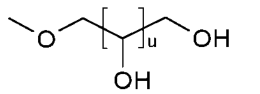

où u représente un nombre entier de la plage de 1 à 6, de préférence 1 à 4,
o' représente un nombre entier de la plage de 1 à 10,
p' représente 1 ou 2,
Ar représente un cycle aromatique non substitué, monosubstitué ou polysubstitué ou un système cyclique condensé comprenant 6 à 18 atomes de carbone, dans lequel un ou deux groupes CH peuvent également être remplacés par C=O,
glycoside représente un glucide éthérifié, de préférence un monoglucoside, un diglucoside, un triglucoside ou un oligoglucoside,
et les groupes amphotères X, $X^1$ et $X^2$ sont choisis parmi les groupes fonctionnels des acétyldiamines, des acides N-alkylaminés, des acides N-alkylaminosulfoniques, des bétaïnes, des sulfobétaïnes ou des dérivés correspondants, en particulier choisis parmi les groupes suivants, où M représente H ou un ion de métal alcalin, de préférence $Li^+$, $Na^+$ ou $K^+$ :

| |
|---|
| 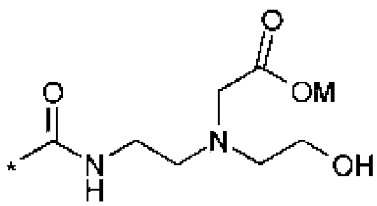 |
| 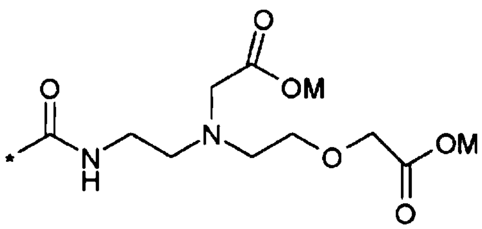 |

(fortgesetzt)

| |
|---|
| 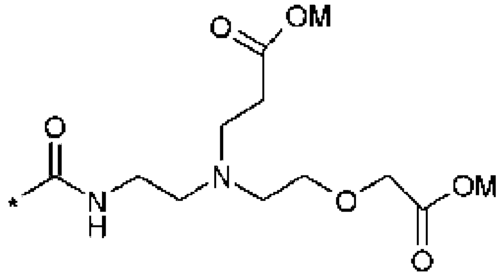 |
| -NH-$CH_2$-COOM ; -NH-$CH_2$-$CH_2$-COOM |
| 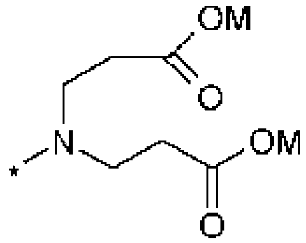 |
| -[(C(=O)-NH-$(CH_2)_{(1-8)}$]$_{(0\ ou\ 1)}$-N+$R^1R^2$-$CH_2$-$COO^-$, où $R^1$ et $R^2$ représentent, à chaque fois indépendamment l'un de l'autre, un radical C1-8-alkyle, de préférence méthyle ou éthyle |
| -C(=O)-NH-$(CH_2)_{1-3}$-$N^+R^1R^2$-$CH_2$-CH(OH)-$CH_2$-$(O)_{(0\ ou\ 1)}$-(S ou P)$O_3^-$, où $R^1$ et $R^2$ représentent, à chaque fois indépendamment l'un de l'autre, un radical C1-8-alkyle, de préférence méthyle ou éthyle. |

**2.** Composés selon la revendication 1, **caractérisés en ce que**

m = 2-4, en particulier 2-3, et
n = 1 ou 2.

**3.** Composés selon l'une ou plusieurs des revendications 1 à 2, **caractérisés en ce que** le groupe fluoré $R^1$ dans la formule (I) est choisi parmi les groupes $CF_3$-$(CF_2)_{0-3}$-, $CF_3$-$(CF_2)_{0-3}$-O-, $CF_3$-$(CF_2)_{0-3}$-O-$(CF_2)_{1-3}$-, $CF_3$-$(CF_2)_{0-3}$-O-$(CF_2)_{1-3}$-O-, $CF_3$-$(CF_2)_{0-3}$-O-$(CF_2)_{1-3}$-O-$CF_2$-, $CF_3$-$(CF_2)_{0-3}$-O-$(CF_2$-$O)_{1-8}$- et $CF_3$-$(CF_2)_{0-3}$-O-(C-$F_2$-$O)_{1-8}$-$CF_2$-.

**4.** Composés selon l'une ou plusieurs des revendications 1 à 3, **caractérisés en ce qu'**ils correspondent à l'une des formules (IIa-c) ou (V) à (VII) :

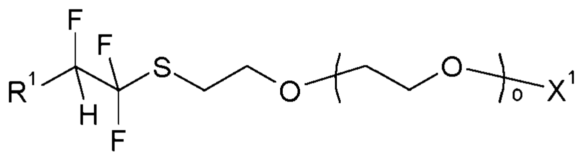

(IIa)

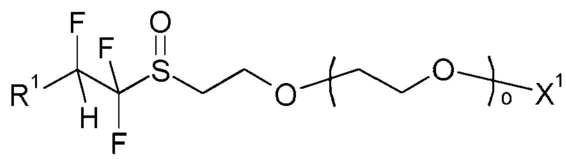

(IIb)

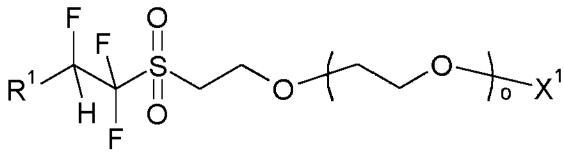

(IIc)

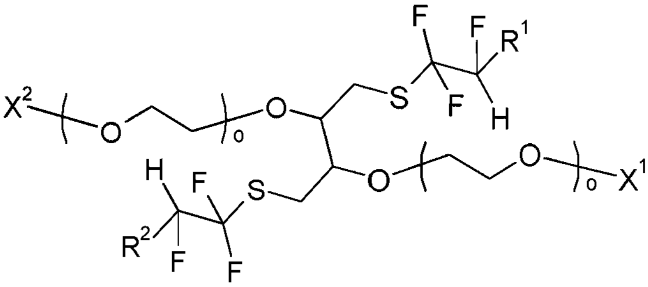

(V)

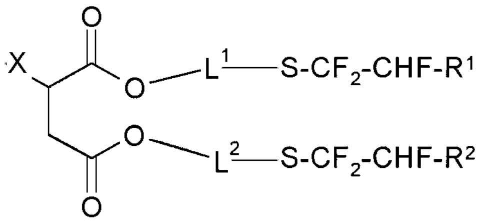

(VI)

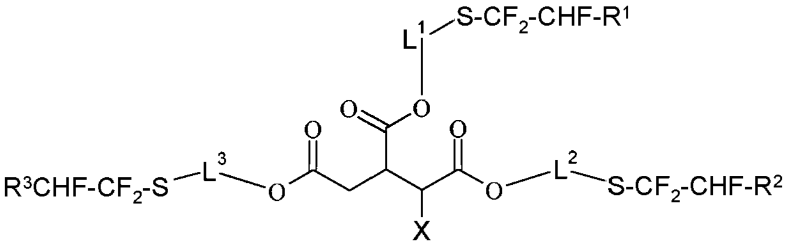

(VII)

où $R^1$, $R^2$ et $R^3$ présentent la signification indiquée dans la revendication 1 ou 3, o = 1-30 et 5-30, en particulier 3, 5, 6, 10, 12, 15, 18, 20 ou 24 ; $X^1$ et $X^2$ représentent, indépendamment l'un de l'autre, un groupe anionique, cationique, non ionique ou amphotère hydrophile tel que défini dans la revendication 1 ou, selon le cas, dans les formules (IIa), (IIb), (IIc) et (V), représentent également H ; $L^1$, $L^2$ et $L^3$ représentent, indépendamment l'un de l'autre, un groupe C1-C6-alkyle linéaire ou ramifié.

**5.** Composés selon l'une ou plusieurs des revendications 1 à 4, **caractérisés en ce qu'**ils correspondent à l'une des formules (XIII) à (XVIII) :

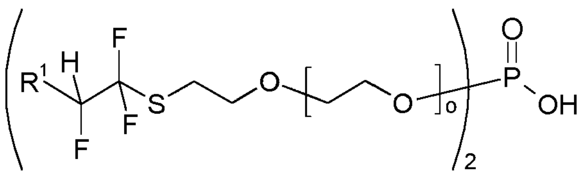

(XIII)

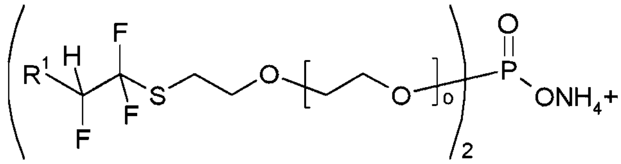

(XIII')

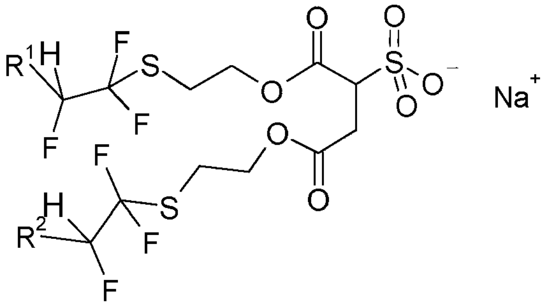

(XIV)

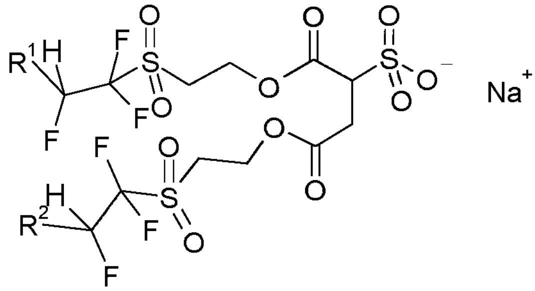

(XIVa)

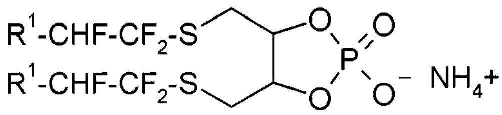

(XV)

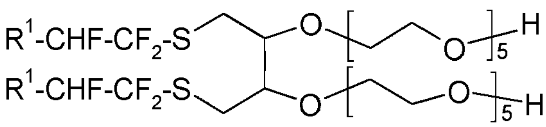

(XVI)

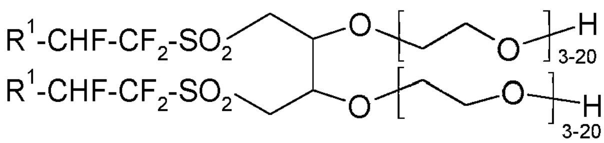

(XVIa)

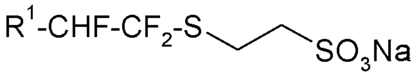

(XVII)

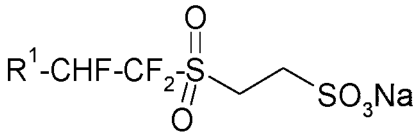

(XVIIa)

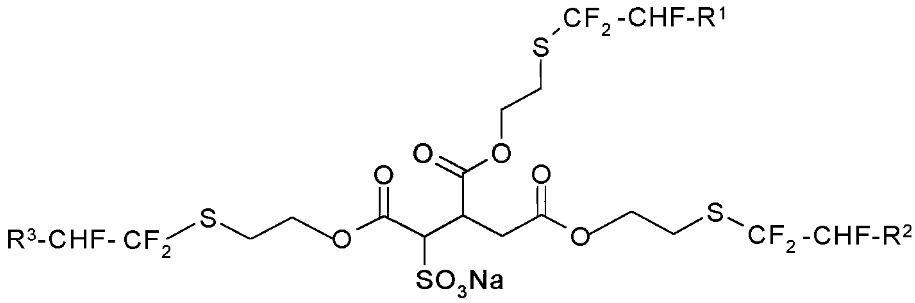

(XVIII)

où $R^1$, $R^2$ et $R^3$ sont choisis, indépendamment les uns des autres, parmi les groupes $CF_3-(CF_2)_{0-3}-$, $CF_3-(CF_2)_{0-3}-O-$, $CF_3-(CF_2)_{0-3}-O-(CF_2)_{1-3}-$, $CF_3-(CF_2)_{0-3}-O-(CF_2)_{1-3}-O-$, $CF_3-(CF_2)_{0-3}-O-(CF_2)_{1-3}-O-CF_2-$, $CF_3-(CF_2)_{0-3}-O-(CF_2-O)_{1-8}-$ et $CF_3-(CF_2)_{0-3}-O-(CF_2-O)_{1-8}-CF_2-$, o vaut 3, 5, 6, 10, 12, 15, 18, 20 ou 24.

**6.** Composés selon l'une ou plusieurs des revendications 1 à 5, **caractérisés en ce qu'**ils correspondent à l'une des formules (XIX) à (XXIX) :

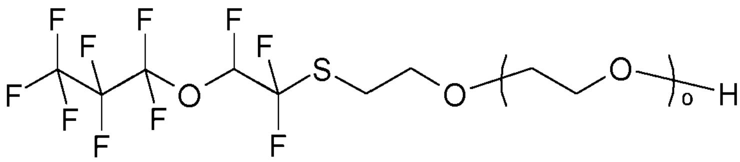

(XIXa)

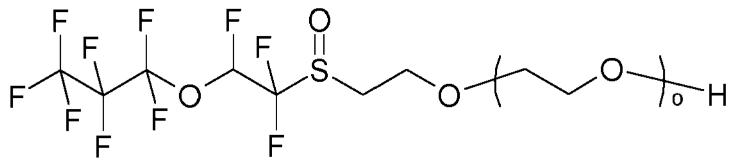

(XIXb)

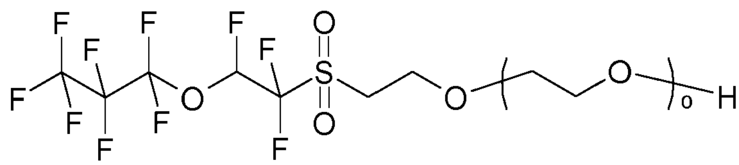

(XIXc)

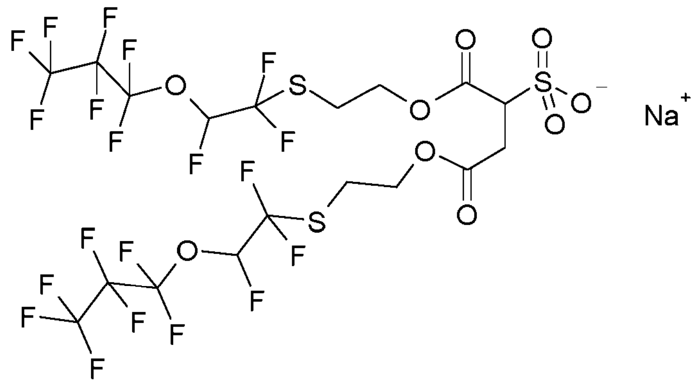

(XX)

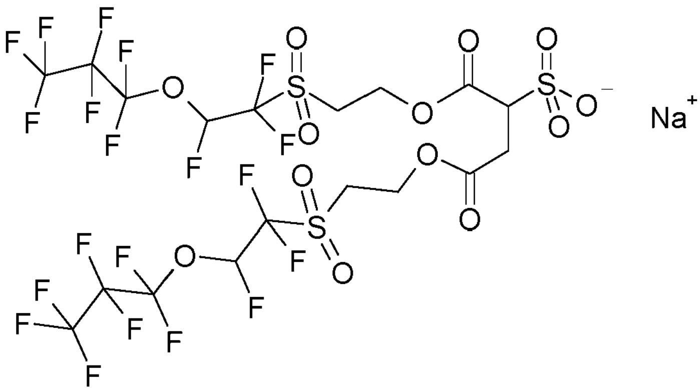

(XXa)

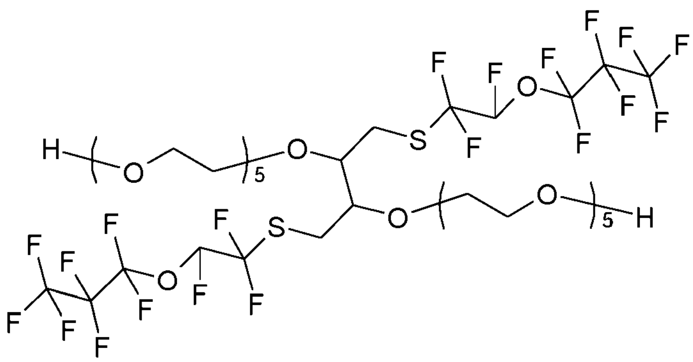

(XXI)

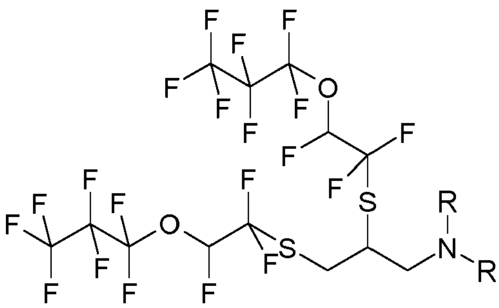

(XXII)

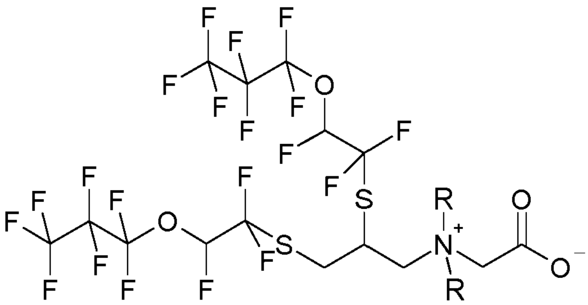

(XXIII)

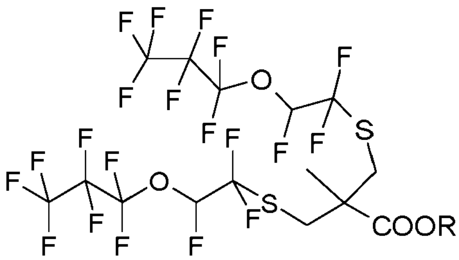

(XXIV)

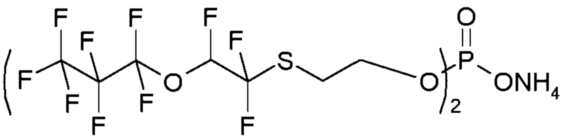

(XXV)

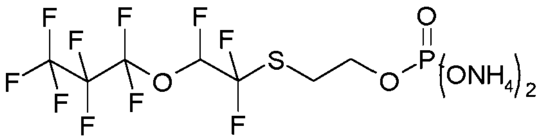

(XXVI)

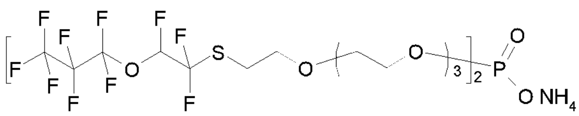

(XXVII)

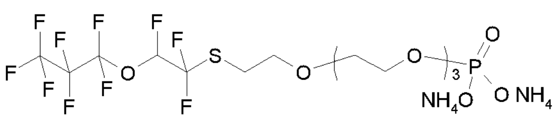

(XXVIII)

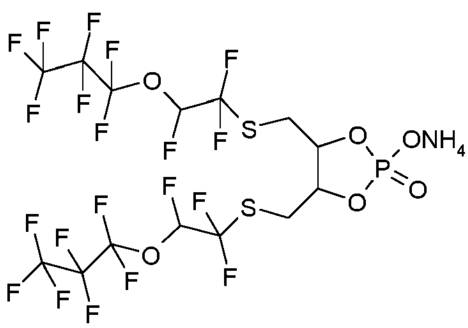

(XXIX)

où o = 0, 10 ou 18 et R = méthyle ou éthyle.

7. Utilisation de composés selon l'une ou plusieurs des revendications 1 à 6 en tant qu'additifs dans les peintures, les laques, les encres d'imprimerie, les enduits de protection, les revêtements spéciaux dans les applications électroniques ou optiques, les laques photosensibles, les revêtements supérieurs antireflet ou les revêtements inférieurs antireflet, les solutions de développement et les solutions de lavage et les résines photosensibles pour les procédés photolithographiques, les produits cosmétiques, les produits agrochimiques, les agents de polissage de sol, les revêtements photographiques ou les revêtements d'éléments optiques.

8. Agent contenant un composé selon l'une ou plusieurs des revendications 1 à 6 et un support approprié pour le but d'utilisation respectif ainsi que le cas échéant d'autres substances actives spécifiques.

9. Agent selon la revendication 8, **caractérisé en ce que** l'agent consiste en des préparations de peinture et de laque, des agents d'extinction d'incendie, des lubrifiants, des agents de lavage et de nettoyage, des dégivreurs, des solutions de développement et des solutions de lavage et des résines photosensibles pour les procédés photolithographiques, des produits cosmétiques, des produits agrochimiques, des agents de polissage de sol ou des agents d'hydrofugation pour l'apprêt de textiles ou le traitement du verre.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2006072401 A
- WO 2010003567 A
- WO 2009149807 A
- WO 2010149262 A
- WO 2011082770 A
- WO 2012084118 A
- WO 2015124290 A
- WO 2016096129 A


### In der Beschreibung aufgeführte Nicht-Patentliteratur


- **G. L. KENNEDY, JR.** ; **J. L. BUTENHOFF** ; **G. W. OLSEN** ; **J. C. O'CONNOR** ; **A. M. SEACAT** ; **R. G. PERKINS** ; **L. B. BIEGEL** ; **S. R. MURPHY** ; **D. G. FARRAR**. *Critical Reviews in Toxicology*, 2004, vol. 34, 351-384